# EUROPEAN PATENT APPLICATION

(11) **EP 4 324 837 A1**
(43) Date of publication of application: **21.02.2024**
(21) Application number: 22787550.7
(22) Date of filing: 13.04.2022
(51) Int. Cl.: C07F 5/02, C07F 5/04, A61K 31/69, A61P 31/00, A61P 31/04

(54) **BORIC ACID DERIVATIVE ACTING AS BETA-LACTAMASE INHIBITOR**

(30) Priority: 13.04.2021 CN 202110392189; 29.07.2021 CN 202110866339; 03.03.2022 CN 202210201742
(71) Applicant: Tuojie Biotech (Shanghai) Co., Ltd., Shanghai 201203 (CN)
(72) Inventor: LIU, Haomiao, Shanghai 201203 (CN); GONG, Honglong, Shanghai 201203 (CN); LI, Wenming, Shanghai 201203 (CN); YU, Jian, Shanghai 201203 (CN); ZHU, Wei, Shanghai 201203 (CN); ZOU, Hao, Shanghai 201203 (CN); LI, Zhengtao, Shanghai 201203 (CN)
(74) Representative: Regimbeau
(86) International application number: PCT/CN2022/086545
(87) International publication number: WO 2022/218328

(57) **Abstract**

A boric acid derivative of a β-lactamase inhibitor as represented by formula I, or a pharmaceutically acceptable salt thereof, and a stereoisomer and a rotamer or a tautomer or a deuterated compound of the derivative and salt. The boric acid derivative can be used to treat bacterial infections.

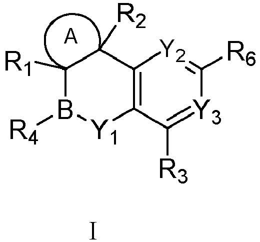

## Description

### TECHNICAL FIELD

The present disclosure belongs to the field of pharmaceutics, and particularly relates to a boric acid derivative as a β-lactamase inhibitor.

### BACKGROUND

β-Lactam antibiotics are antibacterial agents with the widest clinical application and the best anti-infective effect. The most important antibiotics that are currently available are several classes of compounds containing a β-lactam ring, including penicillins, penems, carbapenems, cephalosporins, monobactams and sulfactams. These β-lactam antibiotics inhibit cell wall biosynthesis by binding to proteins called penicillin-binding proteins (PBPs), which are essential for the synthesis of peptidoglycan, a major component of the cell wall of gram-negative and gram-positive bacteria.

β-Lactam antibiotics are still very important worldwide; however, various infectious pathogens have developed resistance to β-lactamase antibiotics, which leads to a reduction in efficacy against bacterial infections. The most significant mechanism of resistance is the production of class A, class C and class D β-lactamases with serine residues at the active center. These enzymes decompose β-lactam antibiotics, causing the loss of antibacterial activity. Class A β-lactamases have substrate specificity mainly for penicillins. Class C β-lactamases have substrate specificity mainly for cephalosporins. As commercially available β-lactamase inhibitors, clavulanic acid, sulbactam and tazobactam are known. These inhibitors are mainly effective against class A β-lactamase-producing bacteria and are used in combination with penicillin-based antibacterial agents. However, more than 250 classes of β-lactamases have been reported to date. In addition to the expansion of class C β-lactamases and extended-spectrum β-lactamases (ESBLs) classified as classes A and D, drug-resistant bacteria which produce class A KPC-2 that decomposes even carbapenems, which are β-lactam-based antibacterial agents of last resort, are also considered a problem.

In recent years, novel β-lactamase inhibitors have been the focus of development, such as QPX-7728 and VNRX-5133. WO2014107536, WO2014089365, WO2018005662 and WO2019226931 disclose a range of β-lactamase inhibitors, with a view to reducing resistance to β-lactamase inhibitors.

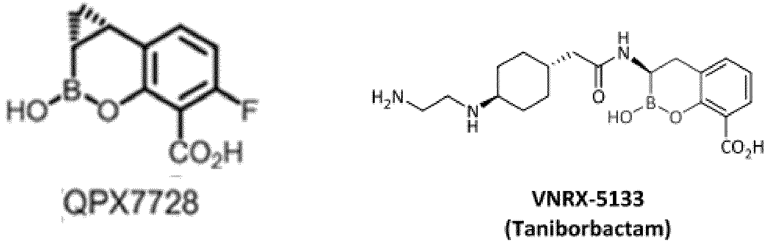

### SUMMARY

The objective of the present disclosure is to provide a boronic acid derivative as a β-lactamase inhibitor, which can be used for treating bacterial infections.

In one aspect, the present disclosure provides a compound of formula (I) or a pharmaceutically acceptable salt thereof, or a stereoisomer, rotamer, tautomer or deuterated compound thereof, 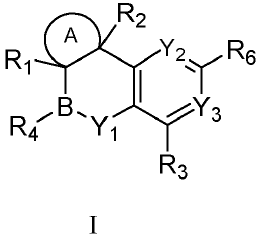 wherein,
ring A is selected from the group consisting of the following optionally substituted ring systems: a carbocycle, a heterocycle, an aromatic ring and a heteroaromatic ring;
Y₁ is selected from the group consisting of -O- and -S-;
Y₂ is selected from the group consisting of CR₅ and N, Y₃ is selected from the group consisting of CR₅' and N, and Y₂ and Y₃ are not simultaneously N;
R₁ and R₂ are each independently selected from the group consisting of hydrogen and the following optionally substituted groups: alkyl, alkenyl, alkynyl, halogen, deuterium, hydroxy, sulfhydryl, - NRiRⱼ, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, alkoxy, alkylthioether group, cycloalkyl, heterocyclyl, aryl and heteroaryl;
R₃ is independently selected from the group consisting of hydrogen and the following optionally substituted groups: alkyl, alkenyl, alkynyl, halogen, deuterium, hydroxy, sulfhydryl, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, alkoxy, alkylthioether group and carboxylic acid isostere;
R₄ is independently selected from the group consisting of the following optionally substituted groups: -NRᵢRⱼ, hydroxy, alkoxy and halogen;
R₅ is selected from the group consisting of hydrogen and the following optionally substituted groups: alkyl, alkenyl, alkynyl, halogen, deuterium, hydroxy, sulfhydryl, -NRᵢRⱼ, -C(O)Rₖ, -C(O)ORₖ, - S(O)Rₖ, -S(O)ORₖ, -S(O)(O)Rₖ, -S(O)(O)ORₖ, -C(S)Rₖ, nitro, cyano, alkoxy, alkylthioether group, cycloalkyl, heterocyclyl, aryl and heteroaryl, or R₅ and R₆, together with their adjacent carbon atoms, form the following optionally substituted ring system: a carbocycle, a heterocycle, an aromatic ring, a heteroaromatic ring, a spirocarbocycle, a spiroheterocycle, a fused carbocycle, a fused heterocycle, a fused aromatic ring or a fused heteroaromatic ring;
R₅' is selected from the group consisting of hydrogen and the following optionally substituted groups: alkyl, alkenyl, alkynyl, halogen, deuterium, hydroxy, sulfhydryl, -NRᵢRⱼ, -C(O)Rₖ, -C(O)ORₖ, - S(O)Rₖ, -S(O)ORₖ, -S(O)(O)Rₖ, -S(O)(O)ORₖ, -C(S)Rₖ, nitro, cyano, alkoxy, alkylthioether group, cycloalkyl, heterocyclyl, aryl and heteroaryl, or R₅' and R₆, together with their adjacent carbon atoms, form the following optionally substituted ring system: a carbocycle, a heterocycle, an aromatic ring, a heteroaromatic ring, a spirocarbocycle, a spiroheterocycle, a fused carbocycle, a fused heterocycle, a fused aromatic ring or a fused heteroaromatic ring;
provided that R₆, at least one of R₅ and R₅', and adjacent carbon atoms form a ring system;
Rᵢ and Rⱼ are each independently selected from the group consisting of hydrogen atom, hydroxy, C₁-C₆ alkyl and C₁-C₆ alkoxy;
Rₖ is independently selected from the group consisting of hydrogen atom, alkyl, haloalkyl, alkoxy, hydroxy and -NRᵢRⱼ, wherein the alkyl, alkoxy and haloalkyl are optionally substituted with one or more substituents selected from the group consisting of alkyl, halogen, hydroxy, sulfhydryl, -NRᵢRⱼ, oxo, thio, carboxyl, nitro, cyano, alkoxy, alkylthioether group, alkenyl, alkynyl, cycloalkyl, heterocyclyl, aryl and heteroaryl.
B is a boron atom.

In certain embodiments, Rₖ is independently selected from the group consisting of hydrogen atom, C₁-C₆ alkyl, C₁-C₆ haloalkyl, C₁-C₆ alkoxy, hydroxy and -NRᵢRⱼ, wherein the alkyl, alkoxy and haloalkyl are optionally substituted with one or more substituents selected from the group consisting of C₁-C₆ alkyl, halogen, hydroxy, sulfhydryl, -NRᵢRⱼ, oxo, thio, carboxyl, nitro, cyano, C₁-C₆ alkoxy, C₁-C₆ alkylthioether group, C₂-C₆ alkenyl, C₂-C₆ alkynyl, 3- to 6-membered cycloalkyl, 3- to 6-membered heterocyclyl, 6- to 10-membered aryl, and 5- to 10-membered heteroaryl.

In certain embodiments, the compound of formula I is a compound of formula I-1 or I-2, 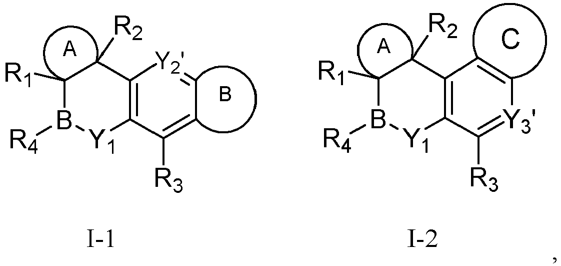 wherein ring B and ring C are each independently selected from the group consisting of the following optionally substituted ring systems: a carbocycle, a heterocycle, an aromatic ring, a heteroaromatic ring, a spirocarbocycle, a spiroheterocycle, a fused carbocycle, a fused heterocycle, a fused aromatic ring and a fused heteroaromatic ring;
Y₂' is selected from the group consisting of CR₅ and N;
Y₃' is selected from the group consisting of CR₅' and N;
R₅ and R₅' are each independently selected from the group consisting of hydrogen and the following optionally substituted groups: alkyl, alkenyl, alkynyl, halogen, deuterium, hydroxy, sulfhydryl, - NRiRⱼ, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, alkoxy, alkylthioether group, cycloalkyl, heterocyclyl, aryl and heteroaryl.

In certain embodiments, the ring system formed from R₆, at least one of R₅ and R₅' and adjacent carbon atoms, ring B or ring C is independently selected from the group consisting of the following ring systems: 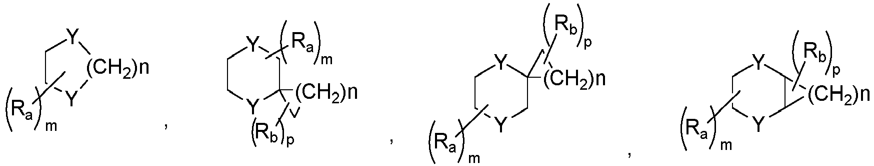 wherein,
Y is independently selected from the group consisting of CH₂, NH, O and S;
Rₐ and R_{b} are each independently selected from the group consisting of the following optionally substituted groups: C₁-C₆ alkyl, halogen, deuterium, hydroxy, sulfhydryl, -NRᵢRⱼ, oxo, thio, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthioether group, C₂-C₆ alkenyl, C₂-C₆ alkynyl, 3- to 6-membered cycloalkyl, 3- to 6-membered heterocyclyl, 7- to 10-membered fused cycloalkyl, 7- to 10-membered fused heterocyclyl, 6- to 10-membered aryl, 5- to 10-membered heteroaryl, 8- to 12-membered fused cycloaryl, and 5-to 12-membered fused heteroaryl;
n is selected from the group consisting of 1, 2, 3, 4, 5 and 6;
m and p are each independently selected from the group consisting of 0, 1, 2, 3, 4, 5 and 6.

In certain embodiments, R₁ and R₂ are each independently selected from the group consisting of hydrogen and the following optionally substituted groups: C₁-C₆ alkyl, C₂-C₆ alkenyl, C₂-C₆ alkynyl, halogen, deuterium, hydroxy, -NRᵢRⱼ and C₁-C₆ alkoxy.

In certain embodiments, ring A is selected from the group consisting of optionally substituted 3- to 12-membered, more preferably 3- to 6-membered, carbocycle and heterocycle.

In certain embodiments, R₃ is independently selected from the group consisting of optionally substituted -C(O)ORₖ and an optionally substituted carboxylic acid isostere.

In certain embodiments, the carboxylic acid isostere may be selected from the group consisting of tetrazole, -SO₃H, -SO₂HNR, -PO₂(R)₂, -PO₃(R)₂, -CONHNHSO₂R, -COHNSO₂R and -CONRCN, wherein R is selected from the group consisting of hydrogen, C₁-C₆ alkyl, C₂-C₆ alkenyl, C₂-C₆ alkynyl, 3- to 6-membered cycloalkyl, 6- to 10-membered aryl, 5- to 10-membered heteroaryl and 3-to 6-membered heterocyclyl.

In certain embodiments, the compound is selected from the group consisting of 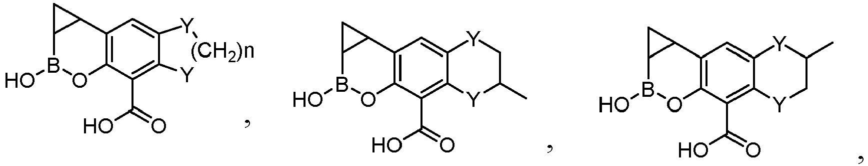 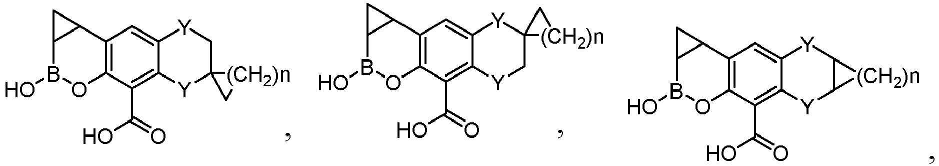 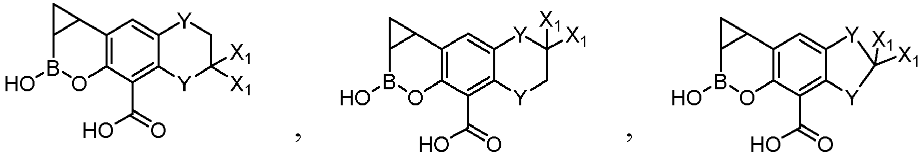
or pharmaceutically acceptable salts thereof, or stereoisomers, rotamers, tautomers or deuterated compounds thereof,
wherein Y is independently selected from the group consisting of CH₂, NH, O and S; n is independently selected from the group consisting of 1, 2, 3, 4, 5 and 6; X₁ is independently selected from the group consisting of F, Cl and Br.

In certain embodiments, the compound is selected from the group consisting of 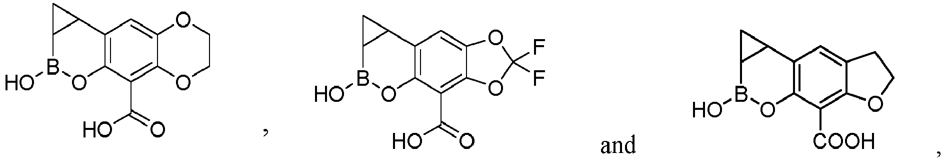 or pharmaceutically acceptable salts thereof, or stereoisomers, rotamers, tautomers or deuterated compounds thereof.

In certain embodiments, the compound is selected from the group consisting of 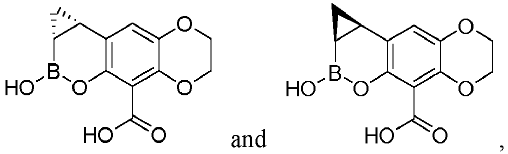 or pharmaceutically acceptable salts thereof, or stereoisomers, rotamers, tautomers or deuterated compounds thereof.

In certain embodiments, the pharmaceutically acceptable salt is selected from the group consisting of an alkali metal salt and an ammonium salt. In certain embodiments, the pharmaceutically acceptable salt is a sodium salt, including a disodium salt.

In certain embodiments, the compound is selected from 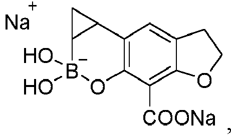 or a stereoisomer, rotamer, tautomer or deuterated compound thereof.

In certain embodiments, the compound is selected from the group consisting of 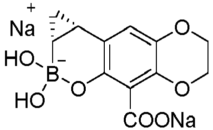 and 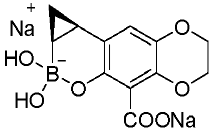 or stereoisomers, rotamers, tautomers or deuterated compounds thereof.

The "alkyl" described herein is preferably C₁-C₆ alkyl.

The "alkenyl" described herein is preferably C₂-C₆ alkenyl.

The "alkynyl" described herein is preferably C₂-C₆ alkynyl.

The "alkylene" described herein is preferably C₁-C₆ alkylene.

The "alkenylene" described herein is preferably C₂-C₆ alkenylene.

The "alkynylene" described herein is preferably C₂-C₆ alkynylene.

The "alkoxy" described herein is preferably C₁-C₆ alkoxy.

The "alkylthioether group" described herein is preferably a C₁-C₆ alkylthioether group.

The "cycloalkyl" described herein is preferably 3- to 12-membered cycloalkyl, and is more preferably 3- to 6-membered cycloalkyl.

The "fused cycloalkyl" described herein is preferably 6- to 14-membered fused cycloalkyl, and is more preferably 7- to 10-membered fused cycloalkyl.

The "heterocyclyl" described herein is preferably 3- to 12-membered heterocyclyl, and is more preferably 3- to 6-membered heterocyclyl.

The "fused heterocyclyl" described herein is preferably 6- to 14-membered fused heterocyclyl, and is more preferably 7- to 10-membered fused heterocyclyl.

The "aryl" described herein is preferably 6- to 14-membered aryl, and is more preferably 6- to 10-membered aryl.

The "fused cycloaryl" described herein is preferably 8- to 14-membered fused cycloaryl, and is more preferably 8- to 12-membered fused cycloaryl.

The "heteroaryl" described herein is preferably 5 to 12-membered heteroaryl, and is more preferably 5 to 10-membered heteroaryl.

The "fused heteroaryl" described herein is preferably 5- to 14-membered fused heteroaryl, and is more preferably 5- to 12-membered fused heteroaryl.

The "optionally substituted" groups described herein may be groups optionally substituted with one or more substituents selected from the group consisting of alkyl (preferably C₁-C₆ alkyl), halogen, deuterium, hydroxy, sulfhydryl, -NRᵢRⱼ, oxo, thio, -C(O)Rₖ, -C(O)ORₖ, -S(O)Rₖ, -S(O)ORₖ, - S(O)(O)Rₖ, -S(O)(O)ORₖ, -C(S)Rₖ, nitro, cyano, alkoxy (preferably C₁-C₆ alkoxy), alkylthioether group (preferably C₁-C₆ alkylthioether group), alkenyl (preferably C₂-C₆ alkenyl), alkynyl (preferably C₂-C₆ alkynyl), cycloalkyl (preferably 3- to 6-membered cycloalkyl), heterocyclyl (preferably 3- to 6-membered heterocyclyl), fused cycloalkyl (preferably 7- to 10-membered fused cycloalkyl), fused heterocyclyl (preferably 7- to 10-membered fused heterocyclyl), aryl (preferably 6- to 10-membered aryl), heteroaryl (preferably 5- to 10-membered heteroaryl), fused cycloaryl (preferably 8- to 12-membered fused cycloaryl), and fused heteroaryl (preferably 5- to 12-membered fused heteroaryl). Rᵢ, Rⱼ and Rₖ are as described above. The set of optional substituents for each "optionally substituted" group may be identical or different.

The present disclosure also provides a pharmaceutical composition comprising at least one of the compounds or the pharmaceutically acceptable salts thereof, or the stereoisomers, rotamers, tautomers or deuterated compounds thereof described above, and a pharmaceutically acceptable carrier, diluent or excipient.

In certain embodiments, a unit dose of the pharmaceutical composition is 0.001-1000 mg.

In certain embodiments, the pharmaceutical composition comprises 0.01%-99.99% of the compound described above based on the total weight of the composition. In certain embodiments, the pharmaceutical composition comprises 0.1%-99.9% of the compound described above. In certain embodiments, the pharmaceutical composition comprises 0.5%-99.5% of the compound described above. In certain embodiments, the pharmaceutical composition comprises 1%-99% of the compound described above. In certain embodiments, the pharmaceutical composition comprises 2%-98% of the compound described above.

In certain embodiments, the pharmaceutical composition comprises 0.01%-99.99% of the pharmaceutically acceptable carrier, diluent or excipient based on the total weight of the composition. In certain embodiments, the pharmaceutical composition comprises 0.1%-99.9% of the pharmaceutically acceptable carrier, diluent or excipient. In certain embodiments, the pharmaceutical composition comprises 0.5%-99.5% of the pharmaceutically acceptable carrier, diluent or excipient. In certain embodiments, the pharmaceutical composition comprises 1%-99% of the pharmaceutically acceptable carrier, diluent or excipient. In certain embodiments, the pharmaceutical composition comprises 2%-98% of the pharmaceutically acceptable carrier, diluent or excipient.

The present disclosure also provides use of the compound or the pharmaceutically acceptable salt thereof, or the stereoisomer, rotamer, tautomer or deuterated compound thereof of the present disclosure for treating a bacterial infection. Examples of bacterial organisms include gram-positive bacteria, gram-negative bacteria, aerobic bacteria and anaerobic bacteria, such as *Staphylococcus, Lactobacillus, Streptococcus, Sarcina, Escherichia, Enterobacter, Klebsiella, Pseudomonas, Acinetobacter, Mycobacterium, Proteus, Campylobacter, Citrobacter, Nisseria, Baccillus, Bacteroides, Peptococcus, Clostridium, Salmonella, Shigella, Serratia, Haemophilus, Brucella* and other organisms.

More examples of bacterial infections include infections with *Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonellaenteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteusmirabilis, Proteus vulgaris, Providenciaalcalifaciens, Providencia rettgeri, Providencia stuartii, Acinetobacter baumannii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilusinfluenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrioparahaemolyticus, Legionella pneumophila, Listeria monocytogenes, Neisseria gonorrhoeae, Neisseria meningitidis, Kingella, Moraxella, Gardnerella vaginalis, Bacteroides fragilis, Bacteroides distasonis, Bacteroides* 3452A homology group, *Bacteroides vulgatus, Bacteroides ovatus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheriae, Corynebacterium ulcerans, Streptococcus pneumoniae, Streptococcus agalactiae, Streptococcus pyogenes, Enterococcus faecalis, Enterococcus faecium, Staphylococcus aureus, Staphylococcus epidermidis, Staphylococcus saprophyticus, Staphylococcus intermedius, Staphylococcus hyicus subsp. hyicus, Staphylococcus haemolyticus, Staphylococcus hominis* or *Staphylococcus saccharolyticus.*

The compound or the pharmaceutically acceptable salt thereof, or the stereoisomer, rotamer, tautomer or deuterated compound thereof of the present disclosure can be used in combination with one or more additional antibiotics for treating a bacterial infection. The additional antibiotics are, for example, β-lactam antibiotics.

The present disclosure further provides a method for treating a bacterial infection in a mammal, which may be a human or a non-human mammal, for therapeutic purposes, the method comprising administering to the mammal the compound or the pharmaceutically acceptable salt thereof, or the stereoisomer, rotamer, tautomer or deuterated compound thereof, or the pharmaceutical composition of the present disclosure.

The present disclosure further provides a kit comprising the compound or the pharmaceutically acceptable salt thereof, or the stereoisomer, rotamer, tautomer or deuterated compound thereof, or the pharmaceutical composition of the present disclosure.

### Terms and definitions:

Unless otherwise stated, the terms used in the specification and claims have the following meanings. The term "alkyl" refers to a saturated aliphatic hydrocarbon group which is a linear or branched group containing 1 to 20 carbon atoms, preferably an alkyl group containing 1 to 12 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, and the like. More preferred is an alkyl group containing 1 to 6 carbon atoms; non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. Alkyl may be substituted or unsubstituted, and when it is substituted, the substituent may be substituted at any available point of attachment, and the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl and carboxylate group.

The term "alkylene" refers to a saturated linear or branched aliphatic hydrocarbon group having 2 residues derived from the parent alkane by removal of two hydrogen atoms from the same carbon atom or two different carbon atoms. It is a linear or branched group containing 1 to 20 carbon atoms, preferably alkylene containing 1 to 12 carbon atoms, and more preferably alkylene containing 1 to 6 carbon atoms. Non-limiting examples of alkylene include, but are not limited to, methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), and the like. Alkylene may be substituted or unsubstituted, and when it is substituted, the substituent may be substituted at any available point of attachment.

The term "alkenylene" refers to a linear alkenyl group having 2 to 8 carbon atoms, preferably 2 to 6 carbon atoms, and more preferably 2 to 4 carbon atoms, and having at least one double bond at any position, including, for example, ethenylene, allylene, propenylene, butenylene, prenylene, butadienylene, pentenylene, pentadienylene, hexenylene, hexadienylene, and the like.

The term "alkynylene" refers to a linear alkynylene group having 2 to 8 carbon atoms, preferably 2 to 6 carbon atoms, and more preferably 2 to 4 carbon atoms, and having at least one triple bond at any position, including, for example, ethynylene, propynylene, butynelene, pentynylene, hexynylene, and the like.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spirocycloalkyl, fused cycloalkyl, and bridged cycloalkyl. "Carbocycle" refers to the ring system in cycloalkyl.

The term "spirocycloalkyl" refers to a 5- to 20-membered polycyclic group in which monocyclic rings share one carbon atom (referred to as the spiro atom). It may contain one or more double bonds, but none of the rings has a fully conjugated π-electron system. It is preferably 6- to 14-membered, and is more preferably 7- to 10-membered. According to the number of spiro atoms shared among the rings, spirocycloalkyl may be monospirocycloalkyl, bispirocycloalkyl or polyspirocycloalkyl. Monospirocycloalkyl and bispirocycloalkyl are preferred. 4-membered/4-membered, 4-membered/5-membered, 4-membered/6-membered, 5-membered/5-membered or 5-membered/6-membered monospirocycloalkyl is more preferred. "Spirocarbocycle" refers to the ring system in spirocycloalkyl. Non-limiting examples of spirocycloalkyl include:

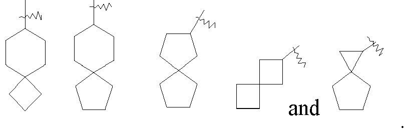

The term "fused cycloalkyl" refers to a 5- to 20-membered all-carbon polycyclic group in which each ring in the system shares a pair of adjacent carbon atoms with other rings in the system, wherein one or more rings may contain one or more double bonds, but none of them has a fully conjugated π-electron system. It is preferably 6- to 14-membered, and is more preferably 7- to 10-membered. According to the number of constituent rings, fused cycloalkyl may be bicyclic, tricyclic, tetracyclic or polycyclic fused cycloalkyl, preferably bicyclic or tricyclic fused cycloalkyl, and more preferably 5-membered/5-membered or 5-membered/6-membered bicycloalkyl. "Fused carbocycle" refers to the ring system in fused cycloalkyl. Non-limiting examples of fused cycloalkyl include:

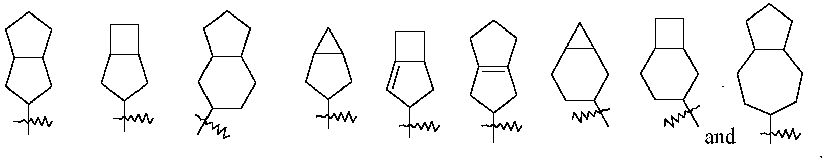

The term "bridged cycloalkyl" refers to a 5- to 20-membered all-carbon polycyclic group in which any two rings share two carbon atoms that are not directly connected to each other. It may contain one or more double bonds, but none of the rings has a fully conjugated π-electron system. It is preferably 6- to 14-membered, and is more preferably 7- to 10-membered. According to the number of constituent rings, bridged cycloalkyl may be bicyclic, tricyclic, tetracyclic or polycyclic bridged cycloalkyl, preferably bicyclic, tricyclic or tetracyclic bridged cycloalkyl, and more preferably bicyclic or tricyclic bridged cycloalkyl. Non-limiting examples of bridged cycloalkyl include:

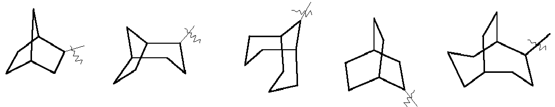

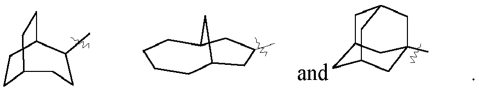

The cycloalkyl ring may be fused to an aryl, heteroaryl or heterocycloalkyl ring, wherein the ring attached to the parent structure is cycloalkyl; non-limiting examples include indanyl, tetrahydronaphthyl, benzocycloheptyl, and the like. Cycloalkyl may be optionally substituted or unsubstituted, and when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl and carboxylate group.

The term "heterocyclyl" refers to a saturated or partially unsaturated monocyclic or polycyclic hydrocarbon substituent containing 3 to 20 ring atoms, one or more of which are heteroatoms selected from the group consisting of nitrogen, oxygen and S(O)ₘ (where m is an integer of 0 to 2), excluding a ring moiety of -O-O-, -O-S- or -S-S-, and the remaining ring atoms are carbon atoms. It preferably contains 3 to 12 ring atoms, of which 1 to 4 are heteroatoms; more preferably, it contains 3 to 6 ring atoms. Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, and the like, preferably piperidinyl and pyrrolidinyl. Polycyclic heterocyclyl includes spiroheterocyclyl, fused heterocyclyl, and bridged heterocyclyl. "Heterocycle" refers to the ring system in heterocyclyl. The term "spiroheterocyclyl" refers to a 5- to 20-membered polycyclic heterocyclyl group in which monocyclic rings share one atom (referred to as the spiro atom), wherein one or more ring atoms are heteroatoms selected from the group consisting of nitrogen, oxygen and S(O)ₘ (where m is an integer of 0 to 2), and the remaining ring atoms are carbon atoms. It may contain one or more double bonds, but none of the rings has a fully conjugated π-electron system. It is preferably 6- to 14-membered, and is more preferably 7- to 10-membered. According to the number of spiro atoms shared among the rings, spiroheterocyclyl may be monospiroheterocyclyl, bispiroheterocyclyl or polyspiroheterocyclyl. Monospiroheterocyclyl and bispiroheterocyclyl are preferred. 4-membered/4-membered, 4-membered/5-membered, 4-membered/6-membered, 5-membered/5-membered or 5-membered/6-membered monospiroheterocyclyl is more preferred. "Spiroheterocycle" refers to the ring system in spiroheterocyclyl. Non-limiting examples of spiroheterocyclyl include:

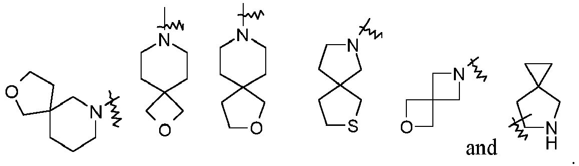

The term "fused heterocyclyl" refers to a 5- to 20-membered polycyclic heterocyclyl group in which each ring shares a pair of adjacent atoms with the other rings in the system, wherein one or more of the rings may contain one or more double bonds, but none of them has a fully conjugated π-electron system, and one or more of the ring atoms are heteroatoms selected from the group consisting of nitrogen, oxygen and S(O)ₘ (where m is an integer of 0 to 2), and the remaining ring atoms are carbon atoms. It is preferably 6- to 14-membered, and is more preferably 7- to 10-membered. According to the number of constituent rings, fused heterocyclyl may be bicyclic, tricyclic, tetracyclic or polycyclic fused heterocyclyl, preferably bicyclic or tricyclic fused heterocyclyl, and more preferably 5-membered/5-membered or 5-membered/6-membered bicyclic fused heterocyclyl. "Fused heterocycle" refers to the ring system in fused heterocyclyl. Non-limiting examples of fused heterocyclyl include:

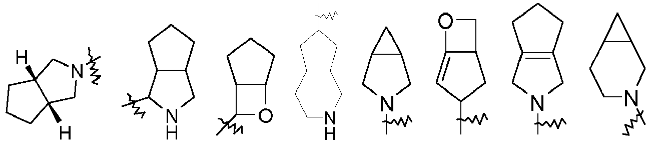

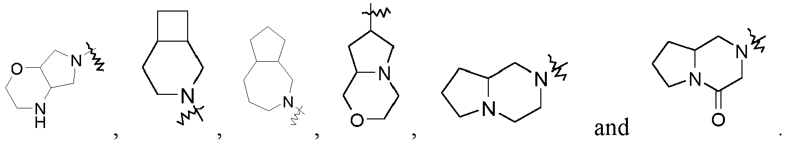

The term "bridged heterocyclyl" refers to a 5- to 14-membered polycyclic heterocyclyl group in which any two rings share two atoms that are not directly connected to each other. It may contain one or more double bonds, but none of the rings has a fully conjugated π-electron system, wherein one or more of the ring atoms are heteroatoms selected from the group consisting of nitrogen, oxygen and S(O)ₘ (where m is an integer of 0 to 2), and the remaining ring atoms are carbon atoms. It is preferably 6- to 14-membered, and is more preferably 7- to 10-membered. According to the number of constituent rings, bridged heterocyclyl may be bicyclic, tricyclic, tetracyclic or polycyclic bridged heterocyclyl, preferably bicyclic, tricyclic or tetracyclic bridged heterocyclyl, and more preferably bicyclic or tricyclic bridged heterocyclyl. Non-limiting examples of bridged heterocyclyl include:

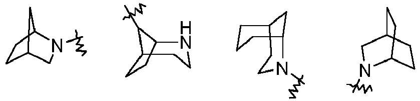

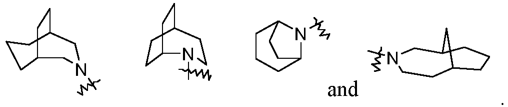

The heterocyclyl ring may be fused to an aryl, heteroaryl or cycloalkyl ring, wherein the ring attached to the parent structure is heterocyclyl; its non-limiting examples include: 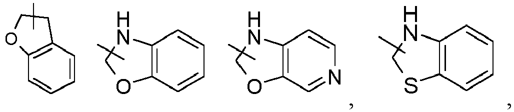 etc.

Heterocyclyl may be optionally substituted or unsubstituted, and when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl and carboxylate group.

The term "aryl" refers to a 6- to 14-membered, preferably 6- to 10-membered all-carbon monocyclic or fused polycyclic (i.e., rings that share a pair of adjacent carbon atoms) group having a conjugated π-electron system, such as phenyl and naphthyl. The aryl ring may be fused to a heteroaryl, heterocyclyl or cycloalkyl ring, wherein the ring attached to the parent structure is the aryl ring. "Aromatic ring" refers to the ring system in aryl. Non-limiting examples of aryl include:

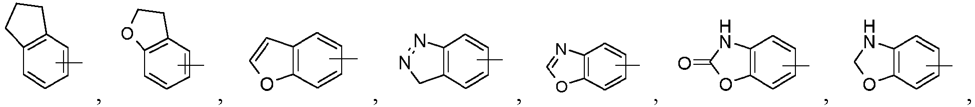

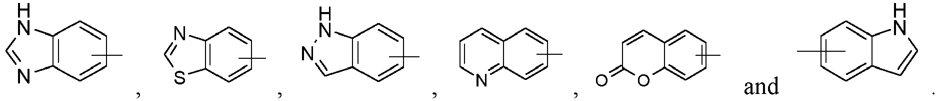

Aryl may be substituted or unsubstituted, and when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl and carboxylate group, preferably phenyl.

The term "fused cycloaryl" may be an unsaturated aromatic fused ring structure containing 8-14 ring atoms, preferably 8-12 ring atoms, formed by connecting two or more ring structures that share two adjacent atoms with each other, including, for example, all unsaturated fused cycloaryl groups such as naphthalene and phenanthrene, and partially saturated fused cycloaryl groups such as benzo 3-8 membered saturated monocyclic cycloalkyl and benzo 3-8 membered partially saturated monocyclic cycloalkyl. "Fused aromatic ring" refers to the ring system in fused cycloaryl. Specific examples of fused cycloaryl include 2,3-dihydro-1H-indenyl, 1H-indenyl, 1,2,3,4-tetrahydronaphthyl, 1,4-dihydronaphthyl, and the like.

The term "heteroaryl" refers to a heteroaromatic system containing 1 to 4 heteroatoms and 5 to 14 ring atoms, wherein the heteroatoms are selected from the group consisting of oxygen, sulfur and nitrogen. Heteroaryl is preferably 5- to 12-membered, e.g., imidazolyl, furyl, thienyl, thiazolyl, pyrazolyl, oxazolyl, pyrrolyl, tetrazolyl, pyridyl, pyrimidinyl, thiadiazole, pyrazinyl, and the like, preferably imidazolyl, pyrazolyl, pyrimidinyl or thiazolyl; and more preferably pyrazolyl or thiazolyl. The heteroaryl ring may be fused to an aryl, heterocyclyl or cycloalkyl ring, wherein the ring attached to the parent structure is the heteroaryl ring. "Heteroaromatic ring" refers to the ring system in heteroaryl. Non-limiting examples of heteroaryl include:

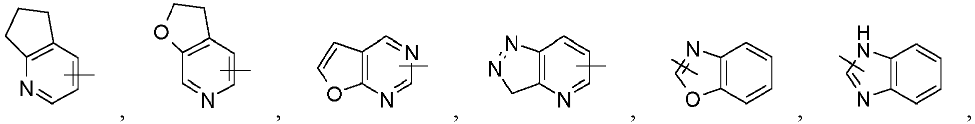

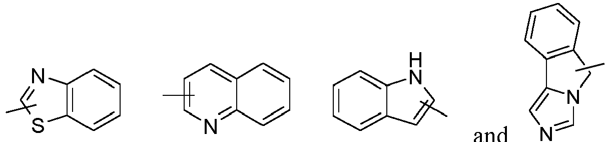

Heteroaryl may be optionally substituted or unsubstituted, and when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl and carboxylate group.

The term "fused heteroaryl" may be an unsaturated aromatic fused ring structure containing 5-14 ring atoms (at least one heteroatom) formed by connecting two or more ring structures that share two adjacent atoms with each other, including the case where a carbon atom, a nitrogen atom and a sulfur atom may be oxidized, preferably "5-12 membered fused heteroaryl", "7-12 membered fused heteroaryl", "9-12 membered fused heteroaryl", and the like, for example, benzofuranyl, benzoisothiafuranyl, benzothienyl, indolyl, isoindolyl, benzoxazolyl, benzimidazolyl, indazolyl, benzotriazolyl, quinolyl, 2-quinolinone, 4-quinolinone, 1-isoquinolinone, isoquinolinyl, acridinyl, phenanthridinyl, benzopyridazinyl, phthalazinyl, quinazolinyl, quinoxalinyl, phenazinyl, pteridinyl, purinyl, naphthyridinyl, phenazine, phenothiazine, and the like. "Fused heteroaromatic ring" refers to the ring system in fused heteroaryl.

Fused heteroaryl may be optionally substituted or unsubstituted, and when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl and carboxylate group.

The term "alkoxy" refers to -O-(alkyl) and -O-(unsubstituted cycloalkyl), wherein the alkyl is as defined above. Non-limiting examples of alkoxy include: methoxy, ethoxy, propoxy, butoxy, cyclopropyloxy, cyclobutoxy, cyclopentyloxy and cyclohexyloxy. Alkoxy may be optionally substituted or unsubstituted, and when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl and carboxylate group.

The term "alkylthio" refers to -S-(alkyl) and -S-(unsubstituted cycloalkyl), wherein the alkyl is as defined above. Non-limiting examples of alkylthio include: methylthio, ethylthio, propylthio, butylthio, cyclopropylthio, cyclobutylthio, cyclopentylthio and cyclohexylthio. Alkylthio may be optionally substituted or unsubstituted, and when it is substituted, the substituent is preferably one or more of the following groups; it is substituted with one or more substituents independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio and heterocycloalkylthio.

The term "hydroxyalkyl" refers to an alkyl group substituted with hydroxy, wherein the alkyl group is as defined above.

The term "haloalkyl" refers to an alkyl group substituted with halogen, wherein the alkyl group is as defined above.

The term "deuterated alkyl" refers to an alkyl group substituted with a deuterium atom, wherein the alkyl group is as defined above.

The term "hydroxy" refers to the -OH group.

The term "oxo" refers to the =O group. For example, a carbon atom is connected to an oxygen atom via a double bond to form a ketone or aldehyde group.

The term "thio" refers to the =S group. For example, the carbon atom is connected to a sulfur atom via a double bond to form thiocarbonyl-C(S)-.

The term "halogen" refers to fluorine, chlorine, bromine or iodine.

The term "amino" refers to -NH₂.

The term "cyano" refers to -CN.

The term "nitro" refers to -NO₂.

The term "carboxyl" refers to -C(O)OH.

The term "aldehyde" refers to -CHO.

The term "carboxylate group" refers to -C(O)O(alkyl) or -C(O)O(cycloalkyl), wherein the alkyl and cycloalkyl are as defined above.

The term "acyl halide" refers to a compound containing a -C(O)-halogen group.

The term "sulfonyl" refers to -S(O)(O)-.

The term "sulfinyl" refers to -S(O)-.

"Isosteres" of a chemical group are other chemical groups that exhibit the same or similar properties. For example, tetrazole is an isostere of carboxylic acid because it mimics the properties of carboxylic acid even though they have very different molecular formulas. Tetrazole is one of many possible isosteric replacements for carboxylic acid. Other carboxylic acid isosteres contemplated include - SO₃H, -SO₂HNR, -PO₂(R)₂, -PO₃(R)₂, -CONHNHSO₂R, -COHNSO₂R and -CONRCN, where R is selected from the group consisting of hydrogen, alkyl, alkenyl, alkynyl, cycloalkyl, aryl, heteroaryl and heterocyclyl as defined herein. In addition, carboxylic acid isosteres may include 5- to 7-membered carbocycles or heterocycles containing any combination of CH₂, O, S or N in any chemically stable oxidation state, where any of the atoms of the ring structures are optionally substituted in one or more positions. It is also contemplated that when chemical substituents are added to a carboxylic isostere, the compound retains the properties of a carboxylic isostere. It is contemplated that when a carboxylic isostere is optionally substituted with one or more moieties selected from R as defined above, the substitution and substitution position are selected such that it does not eliminate the carboxylic acid isosteric properties of the compound. Similarly, it is also contemplated that if one or more R substituents would destroy the carboxylic acid isosteric properties of the compound, the placement of such substituents on a carbocyclic or heterocyclic carboxylic acid isostere is not a substitution at one or more atoms that maintain or are integral to the carboxylic acid isosteric properties of the compound.

"Optional" or "optionally" means that the event or circumstance subsequently described may, but does not necessarily, occur, and that the description includes instances where the event or circumstance occurs or does not occur. For example, "a heterocyclyl group optionally substituted with alkyl" means that the alkyl may, but does not necessarily, exist, and that the description includes instances where the heterocyclyl group is or is not substituted with the alkyl.

"Substituted" means that one or more, preferably up to 5, more preferably 1 to 3 hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort.

In the chemical structure of the compound described herein, a " 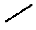 " bond is not specified with a configuration; that is, a " 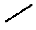 " bond may be " 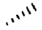 " or " 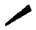 ", or includes both " 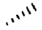 " " and " 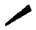 " configurations. In the chemical structure of the compound described herein, a bond " 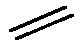 " is not specified with a configuration; that is, it may be in a Z configuration or an E configuration, or contains both configurations.

The present disclosure also includes isotopically-labeled forms of the compound of the present application, which are identical to those described herein but have one or more atoms replaced by an atom having an atomic mass or mass number different from the atomic mass or mass number usually found in nature. Examples of isotopes that can be incorporated into the compound of the present application include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl.

The compound of the present disclosure may contain an unnatural proportion of atomic isotope at one or more of the atoms that constitute the compound. For example, the compound can be labeled with a radioisotope, such as tritium (3H), iodine-125 (125I) or C-14 (¹⁴C). For example, hydrogen may be substituted with deuterium to form a deuterated drug, and the bond formed by deuterium and carbon is firmer than a bond formed by common hydrogen and carbon. The deuterated drug has the advantages of reduced toxic and side effects, increased drug stability, enhanced efficacy, prolonged drug biological half-life period and the like compared with an undeuterized drug. All isotopic variations of the compound of the present application, whether radioactive or not, are intended to be included within the scope of the present application.

Furthermore, substitution with heavier isotopes such as deuterium (i.e., ²H) may provide certain therapeutic advantages (e.g., increased *in vivo* half-life or reduced dose requirement) resulting from greater metabolic stability and hence may be preferred in some circumstances in which deuterium substitution may be partial or complete, wherein partial deuterium substitution refers to substitution of at least one hydrogen with at least one deuterium.

### DETAILED DESCRIPTION

The preparation of the compound described herein and a pharmaceutically acceptable salt thereof is further described below in conjunction with examples, which are not intended to limit the scope of the present disclosure.

Experimental procedures without conditions specified in the examples of the present disclosure are generally conducted according to conventional conditions, or according to conditions recommended by the manufacturers of the starting materials or commercial products. Reagents without specific origins indicated are commercially available conventional reagents.

NMR shifts (δ) are given in 10⁻⁶ (ppm). NMR analysis was performed on a Bruker AVANCE-400 nuclear magnetic resonance instrument, with deuterated dimethyl sulfoxide (DMSO-*d*⁶), deuterated chloroform (CDCl₃), deuterated methanol (CD₃OD) and deuterated acetonitrile (CD₃CN) as solvents and tetramethylsilane (TMS) as an internal standard.

MS analysis was performed on a Shimadzu 2010 Mass Spectrometer or Agilent 6110A MSD Mass Spectrometer.

HPLC analysis was performed on Shimadzu LC-20A systems, Shimadzu LC-2010HT series, or Agilent 1200 LC high-performance liquid chromatograph (Ultimate XB-C18 3.0 × 150 mm chromatography column or Xtimate C18 2.1 × 30 mm chromatography column).

Chiral HPLC analysis used Chiralpak IC-3 100 × 4.6 mm I.D., 3 µm, Chiralpak AD-3 150 × 4.6 mm I.D., 3 µm, Chiralpak AD-3 50 × 4.6 mm I.D., 3 µm, Chiralpak AS-3 150 × 4.6 mm I.D., 3 µm, Chiralpak AS-3 100 × 4.6 mm I.D., 3 µm, ChiralCel OD-3 150 × 4.6 mm I.D., 3 µm, Chiralcel OD-3 100 × 4.6 mm I.D., 3 µm, ChiralCel OJ-H 150 × 4.6 mm I.D., 5 µm, and Chiralcel OJ-3 150 × 4.6 mm I.D., 3 µm chromatography columns. Yantai Huanghai HSGF254 or Qingdao GF254 silica gel plates, 0.15-0.2 mm layer thickness, were adopted for thin-layer chromatography (TLC) analysis and 0.4-0.5 mm layer thickness for TLC separation and purification.

Yantai Huanghai silica gel of 100-200 mesh, 200-300 mesh or 300-400 mesh was generally used as a carrier in column chromatography.

Preparative chiral chromatography used a DAICEL CHIRALPAK IC (250 mm × 30 mm, 10 µm) or Phenomenex-Amylose-1 (250 mm × 30 mm, 5 µm) column.

The CombiFlash preparative flash chromatograph used was CombiFlash Rf150 (TELEDYNE ISCO). The mean inhibition of kinase and the IC₅₀ value were determined on a NovoStar microplate reader (BMG, Germany).

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Pressurized hydrogenation reactions were performed using a Parr 3916EKX hydrogenator and a Qinglan QL-500 hydrogenator, or an HC2-SS hydrogenator.

Hydrogenation reactions generally involved 3 cycles of vacuumization and hydrogen purging.

Microwave reactions were performed on a CEM Discover-S 908860 microwave reactor.

In the examples, a solution was an aqueous solution unless otherwise specified.

In the examples, the reaction temperature was room temperature, i.e., 20 °C to 30 °C, unless otherwise specified.

The monitoring of the reaction progress in the examples was conducted by thin-layer chromatography (TLC). The developing solvent for reactions, the eluent system of column chromatography for compound purification and the developing solvent system of thin-layer chromatography include: A: dichloromethane/methanol system, B: n-hexane/ethyl acetate system, C: petroleum ether/ethyl acetate system, and D: petroleum ether/ethyl acetate/methanol system. The volume ratio of the solvents was adjusted according to the polarity of the compound, or by adding a small amount of basic or acidic reagents such as triethylamine and acetic acid.

The abbreviations used in the following experiments have the following meanings:
DCM: dichloromethane; DIPEA: *N,N*-diisopropylethylamine; CH₃CN: acetonitrile; MeOH: methanol; THF: tetrahydrofuran, NaOH: sodium hydroxide; TsOH: p-toluenesulfonic acid.

### Example 1

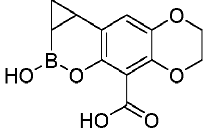

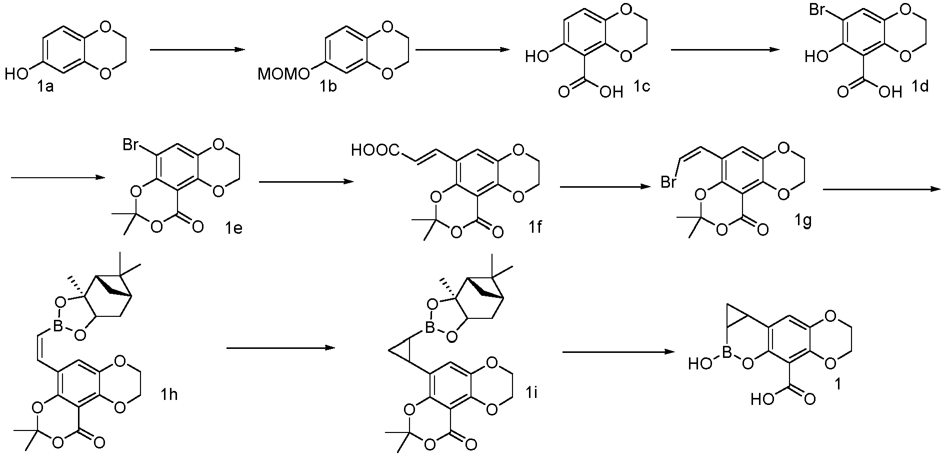

### Step 1

Compound 1a (5 g, 32.86 mmol) was dissolved in tetrahydrofuran (40 mL) in a nitrogen atmosphere, and the solution was cooled to 0 °C. Sodium hydride (2.1 g, 52.50 mmol) was added portionwise. The reaction was stirred, and bromomethyl ether (6.15 g, 49.22 mmol) was added dropwise to the reaction system. After the reaction was complete, the reaction mixture was quenched with water (100 mL), extracted with ethyl acetate, dried over anhydrous sodium sulfate, filtered, and concentrated. The crude product was purified by column chromatography (n-hexane/ethyl acetate = 2/1) and concentrated to give the title compound **1b** (4.39 g, 68% yield).

### Step 2

Compound **1c** (5.0 g, 25.48 mmol) and tetramethylethylenediamine (3.6 g, 31.25 mmol) were dissolved in 50 mL of tetrahydrofuran. After nitrogen purging, the solution was cooled to -78 °C. n-Butyllithium (15 mL, 37.5 mmol) was added dropwise to the reaction, and the reaction was stirred. Crushed dry ice (11 g, 250 mmol) was added to the reaction, and the reaction was stirred, slowly warmed to room temperature and quenched with a 1 mol/L solution of hydrochloric acid (100 mL). Trifluoroacetic acid (3 mL) was added, and the mixture was stirred at room temperature and then extracted with ethyl acetate. The organic phase was dried over anhydrous sodium sulfate, filtered and concentrated. The crude product was purified by column chromatography (dichloromethane/methanol = 10/1) and concentrated to give the title compound **1c** (6.3 g, 100% yield).
MS (ESI) m/z 195.2 [M-H]⁻

### Step 3

Compound **1c** (3.9 g, 19.88 mmol) was dissolved in 40 mL of N,N-dimethylformamide at room temperature, and N-bromosuccinimide (3.18 g, 17.89 mmol) was added portionwise to the reaction. The reaction was stirred at room temperature. Water (50 mL) was added, and the mixture was extracted with ethyl acetate. The organic phase was washed with saturated brine, dried over anhydrous sodium sulfate, filtered and concentrated. The crude product was purified by column chromatography (dichloromethane/methanol = 10/1) and concentrated to give the title compound **1d** (3.9 g, 71.3% yield).
MS (ESI) m/z 275.2, 277.2 [M+H]⁺

### Step 4

Compound **1d** (3.0 g, 10.90 mmol) and trifluoroacetic acid (8 mL) were added to a reaction flask and warmed to 70 °C, and acetone (3.8 g, 65.44 mmol) and trifluoroacetic anhydride (4.6 g, 21.71 mmol) were simultaneously slowly added to the reaction using two syringe pumps. The reaction was stirred at 70 °C, cooled to room temperature after completion, and concentrated. A saturated solution of sodium bicarbonate (150 mL) was added, and the mixture was extracted with ethyl acetate, washed with saturated brine, dried over anhydrous sodium sulfate, filtered, and concentrated. The crude product was purified by column chromatography (n-hexane/ethyl acetate = 1/1) and concentrated to give the title compound **1e** (1.97 g, 57.6% yield). MS (ESI) m/z 315.2, 317.2 [M+H]⁺

### Step 5

Compound **1e** (1.97 g, 6.25 mmol), acrylic acid (0.68 g, 9.38 mmol), palladium acetate (210 mg, 0.94 mmol), triethylamine (1.9 g, 18.75 mmol) and tris(2-tolyl)phosphine (571 mg, 1.89 mmol) were dissolved in N,N-dimethylformamide (8 mL). After nitrogen purging, the reaction was microwaved at 100 °C for 5 h, cooled to room temperature, filtered, and concentrated. The crude product was purified by column chromatography (dichloromethane/methanol = 10/1) to give the title compound **1f** (1.4 g, 73.1% yield). MS (ESI) m/z 307.4 [M+H]⁺

### Step 6

Compound **1f** (1.5 g, 4.90 mmol) was dissolved in 20 mL of chloroform at room temperature. After nitrogen purging, the solution was cooled to 0 °C. Liquid bromine (2.0 mL, 5.36 mmol) was added dropwise to the reaction at 0 °C over 5 min, and the reaction was stirred at 0 °C for 2 h. The reaction mixture was concentrated under reduced pressure and then dissolved in N,N-dimethylformamide (20 mL), and the solution was cooled to 0 °C. Triethylamine (1.19 mL, 8.58 mmol) was added dropwise to the reaction at 0 °C over 2 min, and the reaction was slowly warmed to room temperature and stirred for 12 h. Water (50 mL) was added, and the organic phase was extracted three times with ethyl acetate (50 mL), washed twice with saturated brine (100 mL), dried over anhydrous sodium sulfate, filtered, and concentrated. The crude product was purified by column chromatography (n-hexane/ethyl acetate = 7/3) and concentrated to give the title compound **1g** (310 mg, 18.6% yield). MS (ESI) m/z 340.9, 342.9 [M+H]⁺

### Step 7

Compound **1g** (310 mg, 1.03 mmol), bis(+)-pinanediolato diboron (553 mg, 1.54 mmol), potassium acetate (202 mg, 2.06 mmol) and [1,1'-bis(diphenylphosphino)ferrocene]palladium(II) dichloride (115 mg, 0.15 mmol) were dissolved in 1,4-dioxane (8 mL). After nitrogen purging, the solution was allowed to react at 60 °C for 2 h, cooled to room temperature, filtered, and concentrated. The crude product was purified by column chromatography (elution using n-hexane/ethyl acetate = 1/1) and concentrated to give the title compound **1h** (210 mg, 51% yield). MS (ESI) m/z 441.5 [M+H]⁺

### Step 8

Dichloromethane (3 mL) was added to a 25 mL reaction flask and cooled to -78 °C after nitrogen purging. A solution of diethylzinc in n-hexane (2.2 mL, 2.2 mmol) and diiodomethane (876 mg, 3.26 mmol) were added dropwise to the reaction flask at -78 °C, and the mixture was stirred at -78 °C for 20 min. Compound **1i** (120 mg, 0.27 mmol) was dissolved in dichloromethane (3 mL) and the solution was added dropwise to the reaction over 5 min. After the dropwise addition, the reaction was stirred at -78 °C for 1 h, slowly warmed to room temperature and stirred at room temperature for 18 h, quenched with a saturated solution of ammonium chloride (10 mL), and extracted three times with ethyl acetate (10 mL). The organic phase was dried over anhydrous sodium sulfate, filtered, and concentrated. The crude product was purified by column chromatography (dichloromethane/methanol = 10/1) and concentrated to give the title compound **1i** (52 mg, 42% yield). MS (ESI) m/z 455.5 [M+H]⁺

### Step 9

Compound **1i** (100 mg, 0.22 mmol) was dissolved in 0.5 mL of 1,4-dioxane at room temperature, and a solution of sodium hydroxide (3 mol/L, 0.5 mL) was added. The reaction was stirred. Then the reaction was cooled to 0 °C, and triethylsilane (30.4 mg, 0.26 mmol), isobutylboronic acid (33.7 mg, 0.33 mmol) and trifluoroacetic acid (0.8 mL) were added sequentially. The reaction was slowly warmed to room temperature. After the reaction was complete, the reaction mixture was concentrated under reduced pressure. The resulting crude product was purified using a C18 reversed-phase column [water (0.1% trifluoroacetic acid)/acetonitrile = 1/1] and lyophilized to give the title compound 1 (12.3 mg, 21.3% yield) as a pair of enantiomers.
¹H NMR (400 MHz, DMSO-*d*₆) δ 6.85 (s, 1H), 4.19 (dq, *J* = 8.9, 3.1 Hz, 4H), 4.12 (d, *J=* 9.7 Hz, 1H), 2.16 (td, *J* = 7.9, 4.0 Hz, 1H), 1.24 (ddd, *J=* 10.8, 7.9, 3.3 Hz, 1H), 0.44 (ddd, *J=* 10.4, 8.1, 6.0 Hz, 1H), 0.22 (dt, *J=* 6.5, 3.7 Hz, 1H); MS (ESI) m/z 263.3 [M+H]⁺

### Example 2

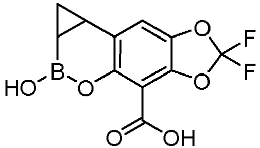

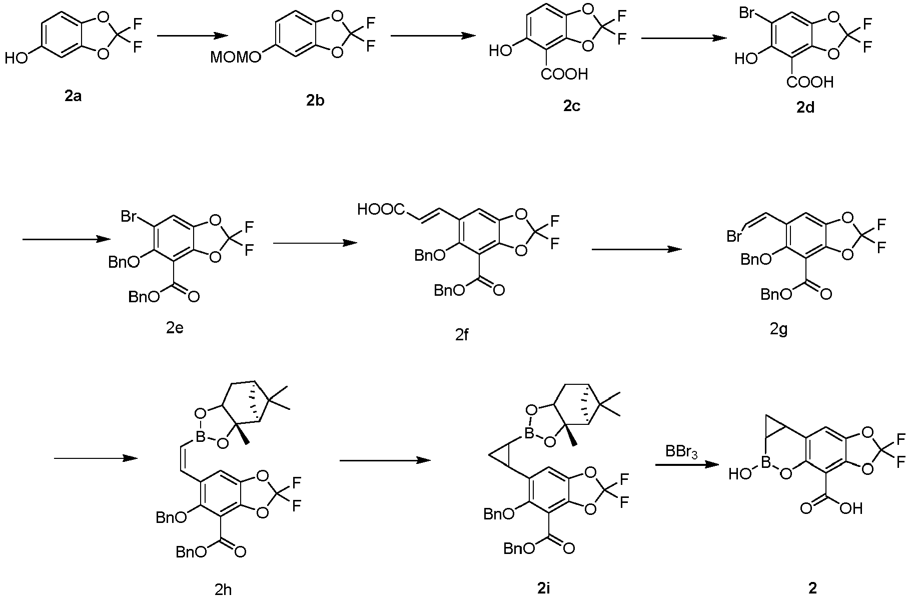

### Step 1

Compound 2a (5 g, 28.7 mmol) was dissolved in tetrahydrofuran (40 mL) in a nitrogen atmosphere, and the solution was cooled to 0 °C. Sodium hydride (1.7 g, 43.0 mmol) was added portionwise, and the reaction was stirred at 0 °C. Then bromomethyl methyl ether (2.8 mL, 34.5 mmol) was added dropwise to the reaction system for a further reaction. After the reaction was complete, the reaction mixture was quenched with water (100 mL), extracted with ethyl acetate, dried over anhydrous sodium sulfate, filtered, and concentrated. The crude product was purified by column chromatography (n-hexane/ethyl acetate = 3/1) and concentrated to give the title compound **2b** (4.5 g, 72% yield). MS (ESI) m/z 219.3 [M+H]⁺

### Step 2

Compound **2b** (4.5 g, 20.6 mmol) and tetramethylethylenediamine (4.6 mL, 30.9 mmol) were dissolved in 50 mL of tetrahydrofuran. After nitrogen purging, the solution was cooled to -78 °C. n-Butyllithium (13.2 mL, 33.0 mmol) was added dropwise to the reaction, and the reaction was stirred. Crushed dry ice (11 g, 250 mmol) was added to the reaction, and the reaction was stirred at -78 °C, then slowly warmed to room temperature and quenched with a 1 mol/L solution of hydrochloric acid (100 mL). Trifluoroacetic acid (3 mL) was added, and the mixture was stirred at room temperature and extracted with ethyl acetate. The organic phase was dried over anhydrous sodium sulfate, filtered and concentrated. The crude product was purified by column chromatography (dichloromethane/methanol = 10/1) and concentrated to give the title compound **2c** (2.5 g, 55% yield). MS (ESI) m/z 217.2 [M-H]⁻

### Step 3

Compound **2c** (2.5 g, 11.5 mmol) was dissolved in 40 mL of DCM at room temperature, and N-bromosuccinimide (2.2 g, 12.6 mmol) was added portionwise to the reaction. The reaction was stirred at room temperature. After the reaction was complete, water (50 mL) was added, and the mixture was extracted with ethyl acetate, washed with saturated brine, dried over anhydrous sodium sulfate, filtered, and concentrated. The crude product was purified by column chromatography (dichloromethane/methanol = 10/1) and concentrated to give the title compound **2d** (2.8 g, 82% yield).
MS (ESI) m/z 296.2, 297.2 [M+H]⁺

### Step 4

Intermediate **2d** (2.8 g, 9.4 mmol) was dissolved in 20 mL of THF, and sodium hydride (0.56 mL, 28.3 mmol) was added under an ice-water bath condition. The reaction was stirred. BnBr (3.4 mL, 28.3 mmol) was slowly added to the reaction mixture, and the stirring was continued. After the reaction was complete, a saturated aqueous solution of ammonium chloride was added, and the mixture was extracted with ethyl acetate, dried over anhydrous sodium sulfate, concentrated under reduced pressure to remove the solvent, and purified by column chromatography (n-hexane/ethyl acetate = 7/1) to give compound **2e** (2.0 g, 44% yield). MS (ESI) m/z 477.2, 479.2 [M+H]⁺.

### Step 5

Compound **2e** (2 g, 4.2 mmol), acrylic acid (0.9 mL, 13.8 mmol), palladium acetate (0.1 g, 0.46 mmol), triethylamine (3.8 mL, 27.7 mmol) and tris(2-tolyl)phosphine (0.28 g, 0.92 mmol) were dissolved in N,N-dimethylformamide (8 mL). After nitrogen purging, the reaction was microwaved at 100 °C, cooled to room temperature, filtered, and concentrated. The crude product was purified by column chromatography (dichloromethane/methanol = 10/1) and concentrated to give the title compound **2f** (2.1 g, 54% yield). MS (ESI) m/z 469.4 [M+H]⁺

### Step 6

Compound **2f** (1.1 g, 2.4 mmol) was dissolved in 20 mL of chloroform at room temperature. After nitrogen purging, the solution was cooled to 0 °C. Liquid bromine (1.0 mL, 2.6 mmol) was added dropwise to the reaction at 0 °C, and the reaction was stirred at 0 °C. The reaction mixture was concentrated under reduced pressure and then dissolved in N,N-dimethylformamide (20 mL). The solution was cooled to 0 °C, and triethylamine (0.6 mL, 4.3 mmol) was added dropwise to the reaction. The reaction was slowly warmed to room temperature and stirred. After the reaction was complete, water (50 mL) was added, and the organic phase was extracted with ethyl acetate, washed with saturated brine, dried over anhydrous sodium sulfate, filtered, and concentrated. The crude product was purified by column chromatography (n-hexane/ethyl acetate = 10/1) and concentrated to give the title compound **2g** (400 mg, 32% yield). MS (ESI) m/z 503.2, 505.2 [M+H]⁺

### Step 7

Compound **2g** (400 mg, 0.79 mmol), bis(+)-pinanediolato diboron (427 mg, 1.19 mmol), potassium acetate (156 mg, 1.59 mmol) and [1,1'-bis(diphenylphosphino)ferrocene]palladium(II) dichloride (117.9 mg, 0.16 mmol) were dissolved in 1,4-dioxane (8 mL). After nitrogen purging, the solution was allowed to react at 60 °C. After the reaction was complete, the reaction mixture was cooled to room temperature, filtered and concentrated. The crude product was purified by column chromatography (n-hexane/ethyl acetate = 2/1) and concentrated to give the title compound **2h** (300 mg, 62% yield). MS (ESI) m/z 603.4 [M+H]⁺

### Step 8

Dichloromethane (6 mL) was added to a 25 mL reaction flask and cooled to -78 °C after nitrogen purging. A solution of diethylzinc in n-hexane (19.9 mL, 19.9 mmol) and diiodomethane (2.68 mL, 33.2 mmol) were added dropwise to the reaction flask at -78 °C, and the reaction was stirred at - 78 °C. Compound **2h** (400 mg, 0.66 mmol) was dissolved in dichloromethane (6 mL) and the solution was added dropwise to the reaction. The reaction was stirred at -78 °C and then slowly warmed to room temperature. After the reaction was complete, the reaction mixture was quenched with a saturated solution of ammonium chloride (10 mL), extracted with ethyl acetate, dried over anhydrous sodium sulfate, filtered, and concentrated. The crude product was purified by column chromatography (dichloromethane/methanol = 9/1) and concentrated to give the title compound **2i** (40 mg, 9.7% yield). MS (ESI) m/z 617.3 [M+H]⁺

### Step 9

Compound **2i** (10 mg, 0.016 mmol) was dissolved in 2 mL of dichloromethane at room temperature, and a solution of boron tribromide in dichloromethane (0.2 mL, 1 M) was added. The mixture was stirred at -78 °C and then slowly warmed to room temperature. After the reaction was complete, the reaction mixture was quenched with methanol and concentrated under reduced pressure. The resulting crude product was purified using a C18 reversed-phase column (water (0.1% trifluoroacetic acid)/acetonitrile = 1/1) and lyophilized to give the title compound **2** (2 mg, 43% yield) as a pair of enantiomers.
¹H NMR (400 MHz, DMSO-*d₆*) δ 6.83 (s, 1H), 2.20-2.24 (m, 1H), 1.21-1.15 (m, 1H), 0.75-0.96(m, 2H); MS (ESI) m/z 285.5 [M+H]⁺

### Example 3

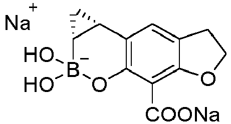

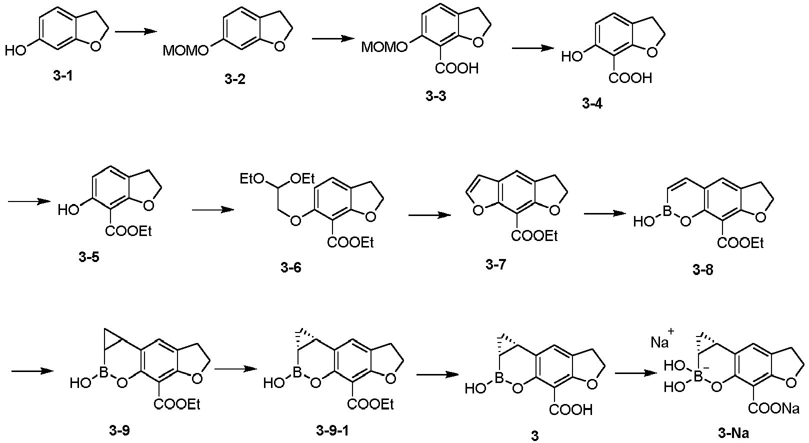

### Step 1

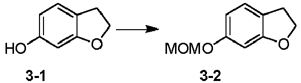

250 mL of DMF was added to a reaction flask, NaH (15.6 g, 3900 mmol, 1.8 eq, 60% wt) was added portionwise in a N₂ atmosphere, and a solution of **3-1** (29.5 g, 216.9 mmol, 1.0 eq) (prepared according to J. Am. Chem. Soc. 1948, 70, 3619) in DMF (50 mL) was slowly added dropwise under an ice bath condition. After the dropwise addition, the mixture was stirred in the ice bath for 10 min. MOMBr (43.4 g, 347 mmol, 1.6 eq) was slowly added dropwise. After the dropwise addition, the mixture was naturally warmed to room temperature and stirred until the reaction was complete. The reaction mixture was quenched by slow addition of water and diluted with 250 mL of ethyl acetate and 250 mL of water. The aqueous phase was separated, extracted with ethyl acetate, washed with a saturated solution of NaCl, dried over anhydrous sodium sulfate, and concentrated *in vacuo* to give a crude product. The crude product was purified by silica gel column chromatography to give **3-2** (about 25.8 g).

¹H NMR (400 MHz, CDCl₃) *δ* 7.07-7.05 (d, *J* = 8.4 Hz, 1H), 6.53-6.51 (m, 2H), 5.13 (s, 2H), 4.59-4.55 (t, *J* = 8.8 Hz, 2H), 3.47 (s, 3H), 3.16-3.12 (t, *J* = 8.8 Hz, 2H).

### Step 2

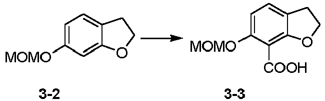

**3-2** (25.8 g, 143 mmol, 1.0 eq) and TMDPA (20.0 g, 172 mmol, 1.2 eq) were added to a reaction flask. After nitrogen purging, anhydrous THF (258 mL, 10 V) was added, and the system was cooled to below -65 °C. 1.6 M n-BuLi (143 mL, 229 mmol, 1.6 eq) was added dropwise in a N₂ atmosphere with the temperature controlled at below -65 °C. After the dropwise addition, the mixture was stirred at -65 °C for 1 h. Dry ice (20 eq) was washed with anhydrous THF and then added portionwise to the reaction mixture, with the reaction temperature controlled at below -30 °C. The reaction was quenched with 200 mL of a saturated solution of citric acid under an ice bath condition. The aqueous phase was separated and extracted with EA/THF (1:1) (150 mL × 3). The organic phases were combined, washed with a saturated solution of NaCl (150 mL), dried over anhydrous sodium sulfate, and concentrated *in vacuo* to give a crude product. The crude product was triturated with 400 mL of PE/EA (3:1) for 1 h, and the triturate was filtered to give **3-3** (about 24.4 g, 76.0% yield).

¹H NMR (400 MHz, DMSO-d⁶) *δ* 12.82 (br, 1H), 7.19-7.16 (d, *J* = 8.4 Hz, 1 H), 6.60-6.58 (d, *J* = 8.4 Hz, 1 H), 5.14 (s, 2H), 4.59-4.55 (t, *J* = 8.8 Hz, 2H), 3.37 (s, 3H), 3.14-3.09 (t, *J* = 8.8 Hz, 2H).

### Step 3

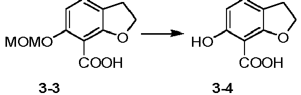

**3-3** (22.2 g, 98.8 mmol, 1.0 eq) was weighed into a reaction flask, EtOH (330 mL, 15 V) was added, and TfOH (23.7 g, 158 mmol, 1.6 eq) was added under an ice bath condition. The mixture was stirred in the ice bath for 20 min. The reaction mixture was diluted with 300 mL of ethyl acetate and 300 mL of a saturated solution of NaCl. The aqueous phase was separated and extracted with ethyl acetate.

The organic phases were combined, dried over anhydrous sodium sulfate, and concentrated to dryness by rotary evaporation *in vacuo* to give crude **3-4.**

¹H NMR (400 MHz, DMSO-d⁶) *δ* 11.48 (br, 2H), 7.28-7.26 (d, *J* = 8.0 Hz, 1 H), 6.36-6.34 (d, *J* = 8.0 Hz, 1 H), 4.63-4.58 (t, *J* = 8.8 Hz, 2H), 3.09-3.05 (t, *J* = 8.8 Hz, 2H).

### Step 4

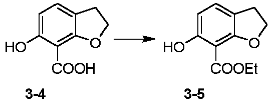

**3-4** (26.0 g, 144 mmol, 1.0 eq) was weighed into a reaction flask, EtOH (260 mL, 10 V) was added, and TfOH (26.0 g, 173 mmol, 1.2 eq) was added. The mixture was refluxed overnight. An additional 260 mL of EtOH was added, and the overnight refluxing was continued. The reaction mixture was diluted with EtOAc (300 mL) and a saturated solution of NaCl (300 mL). The aqueous phase was separated and extracted with EtOAc (200 mL × 2). The organic phases were combined, dried over anhydrous sodium sulfate, filtered, and concentrated *in vacuo* to give a crude product. The crude product was purified by column chromatography to give **3-5** (about 13.2 g, 64% yield over two steps). ¹H NMR (400 MHz, DMSO-d⁶) *δ* 10.60 (br, 1H), 7.25-7.23 (d, *J* = 8.0 Hz, 1 H), 6.38-6.36 (d, *J* = 8.0 Hz, 1 H), 4.63-4.59 (t, *J* = 8.8 Hz, 2H), 4.34-4.29 (q, *J* = 7.2 Hz,2H), 3.10-3.05 (t, *J* = 8.8 Hz, 2H), 1.31-1.27 (t, *J* = 7.2 Hz, 3H).

### Step 5

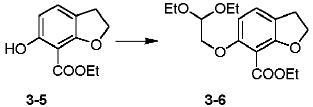

Compound **3-5** (13.2 g, 63.5 mmol, 1.0 eq) was weighed into a reaction flask, DMF (132 mL, 10 V) and t-BuOK (9.26 g, 82.5 mmol, 1.3 eq) were added, and 2-bromo-1,1-diethoxyethane (15.0 g, 76.2 mmol, 1.2 eq) was added dropwise under an ice bath condition. After the dropwise addition, the mixture was warmed to 140 °C and stirred overnight. The reaction mixture was diluted with EtOAc (200 mL) and H₂O (200 mL). The aqueous phase was separated and extracted with EtOAc (100 mL × 2). The organic phases were combined, washed with H₂O (200 mL × 2) and then with a saturated solution of NaCl (200 mL), dried over anhydrous sodium sulfate, filtered, and concentrated *in vacuo* to give a crude product. The crude product was purified by silica gel column chromatography to give **3-6** (about 9.00 g, 43.8% yield).

¹H NMR (400 MHz, CDCl₃) *δ* 7.12-7.09 (m, 1H), 6.39-6.37 (d, *J* = 8.4 Hz, 1H), 4.81-4.78 (t, *J=* 5.2 Hz, 1H), 4.67-4.62 (t, *J* = 8.8 Hz, 2H), 4.39-4.33 (q, *J* = 7.2 Hz, 2H), 4.00-3.99 (d, *J* = 5.2 Hz, 2H), 3.79-3.71 (m, 2H), 3.66-3.58 (m, 2H), 3.16-3.11 (m, 2H), 1.39-1.35 (t, *J* = 14.0 Hz, 3H), 1.25-1.21 (t, *J* = 6.8 Hz, 6H).

### Step 6

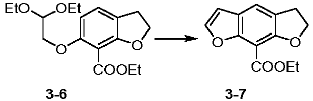

**3-6** (9.80 g, 30.2 mmol, 1.0 eq), 98 mL of DCE (10 V) and 2.38 g of Amberlyst 15 (25% wt) were added to a reaction flask, and the mixture was refluxed until the reaction was complete. The reaction mixture was filtered under reduced pressure, and the filtrate was concentrated under reduced pressure. The crude product was purified by silica gel column chromatography to give **3-7** (about 4.51 g, 64.3% yield).

¹H NMR (400 MHz, CDCl₃) *δ* 7.62-7.61 (d, J = 2.0Hz, 1H), 7.47-7.46 (m, 1H), 6.67-6.66 (d, *J* = 2.4 Hz, 1H), 4.80-4.76 (t, *J* = 8.4 Hz, 2H), 4.50-4.51 (q, *J* = 6.8 Hz, 2H), 3.30-3.25 (m, 2H), 1.46-1.42 (t, *J* = 7.2 Hz, 3H).

### Step 7

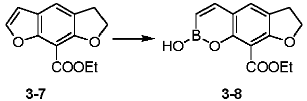

Nickel chloride (0.505 g, 3.88 mmol, 0.2 eq) was weighed into a reaction flask, tetrahydrofuran (22.5 mL, 5 V) was added, and tri-n-octylphosphine (3.20 g, 7.78 mmol, 0.4 eq) was then added. After nitrogen purging, the reaction was refluxed for 1 h and then cooled to room temperature. **3-7** (4.51 g, 19.4 mmol, 1.0 eq) was weighed into another reaction flask, tetrahydrofuran (67.5 mL, 15 V) was added, bis(pinacolato)diboron (7.41 g, 29.2 mmol, 1.5 eq), potassium carbonate (7.24 g, 54.5 mmol, 2.7 eq) and cesium carbonate (1.90 g, 5.83 mmol, 0.3 eq) were then added, and the catalytic system prepared in nickel chloride/tri-n-octylphosphine was finally added. After nitrogen purging, the reaction was refluxed for 2 h. The reaction system was cooled to 0-5 °C, MTBE (199 mL, 15 V) and deionized water (199 mL, 15 V) were added, and the pH was adjusted to 1 with 6 N hydrochloric acid. The mixture was stirred at 0-5 °C for 30 min. The mixture separated into layers. The aqueous phase was extracted once with MTBE (132 mL, 10 V). The organic phases were combined, dried over anhydrous sodium sulfate, filtered under reduced pressure, and concentrated *in vacuo* to give a crude product. The crude product was purified using a preparative C18 reversed-phase column. The positive component was collected, concentrated, adjusted to pH 1-2 with 6 N hydrochloric acid, and then extracted with methyl tert-butyl ether. The organic phase was dried over anhydrous sodium sulfate, filtered and concentrated to dryness to give **3-8** (4.14 g, 82.1% yield).

¹H NMR (400 MHz, DMSO-d⁶) *δ* 8.99 (br, 1H), 7.78-7.75 (d, *J* = 12.0 Hz, 1 H), 7.48 (s, 1H), 6.00-5.97 (d, *J* = 12.0 Hz, 1 H), 4.73-4.69 (t, *J* = 17.2 Hz, 2 H), 4.39-4.33 (q, *J* = 7.2 Hz, 2 H), 3.28-3.24 (t, *J* = 8.8 Hz, 2 H), 1.37-1.33 (t, *J* = 7.2 Hz, 3 H).

### Step 8

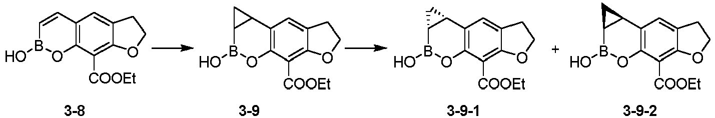

A reaction flask was purged with argon, and a 1 M solution of diethyl zinc in n-hexane (55.7 mL, 4.0 eq) was added. The reaction system was cooled to -40 °C. Diiodomethane (29.8 g, 111 mmol, 8.0 eq) was dissolved in 9 mL of dichloromethane, and the resulting solution was slowly added dropwise to the solution of diethyl zinc in n-hexane using a syringe. The mixture was stirred at -40 °C for another 30 min. Trifluoroacetic acid (6.35 g, 55.7 mmol, 4.0 eq) was dissolved in 9 mL of dichloromethane, and the resulting solution was slowly added dropwise to the above solution using a syringe. The mixture was stirred at -40 °C for another 30 min. **3-8** (3.62 g, 13.9 mmol, 1.0 eq) was dissolved in 12.5 mL of dichloromethane, and the resulting solution was slowly added dropwise to the above system using a syringe. After the dropwise addition, the mixture was slowly warmed to room temperature for another 3 h of reaction. The reaction was quenched with 1 M hydrochloric acid, and the system was diluted with 25 mL of dichloromethane. The organic phase was separated, and the aqueous phase was extracted with 25 mL of dichloromethane. The organic phases were combined, dried over anhydrous sodium sulfate, concentrated *in vacuo* and purified by preparative HPLC to give **3-9** (1.42 g, 37.3% yield). Racemate **3-9** was chirally resolved into **3-9-1** (about 320 mg) and **3-9-2** (about 300 mg).

### Single-configuration compound 3-9-1:

Chiral HPLC analysis: retention time 2.832 min, chiral purity > 99% (column: ChiralPak IG, 250 × 30 mm I.D., 10 µm; mobile phases: A: CO₂; B: Methanol (0.1% NH_{3·}H₂O))

### Single-configuration compound 3-9-2:

Chiral HPLC analysis: retention time 2.968 min, chiral purity > 99% (column: ChiralPak IG, 250 × 30 mm I.D., 10 µm; mobile phases: A: CO₂; B: Methanol (0.1% NH_{3·}H₂O))
Racemate **3-9** ¹H NMR (400 MHz, CDCl₃) *δ* 7.16 (s, 1H), 5.97 (br, 1H), 4.66-4.57 (m, 2H), 4.40-4.34 (q, *J* = 7.2 Hz, 2H), 3.16-3.11 (t, *J* = 8.8 Hz, 2H), 2.18-2.13 (m, 1H), 1.38-1.35(t, *J* = 6.8 Hz, 3H), 1.31-1.25 (m, 1H). 0.61-0.51 (m, 1H), 0.42-0.39 (m, 1H).

### Step 9

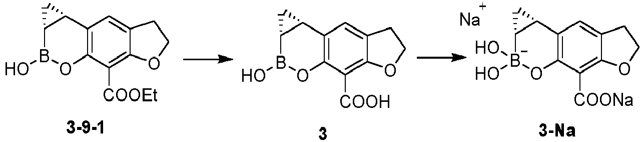

To a reaction flask, 0.25 mL of methanol and 0.25 mL of 1,4-dioxane were added, **3-9-1** (54.8 mg, 0.200 mmol, 1.0 eq) was added, and 0.25 mL of a 25% wt aqueous solution of sodium hydroxide was added. The reaction was left at 50 °C overnight. The reaction mixture was concentrated *in vacuo* to remove methanol and 1,4-dioxane, and an additional 1 mL of deionized water was added. The system was adjusted to pH 8 with 6 M hydrochloric acid and extracted with MTBE. The aqueous phase was adjusted to pH 1-2 with 6 M hydrochloric acid. The aqueous phase was extracted with 5 mL of ethyl acetate until the aqueous phase contained no product. The organic phases were combined, dried over anhydrous sodium sulfate, filtered, and concentrated *in vacuo* to give free acid **3** (40.8 mg). The free acid was dissolved in 4 mL of acetonitrile. 2.0 eq of sodium hydroxide was accurately weighed out to prepare a 4 mL aqueous solution, and the solution was added to the above reaction system. The mixture was stirred at room temperature for 30 min. The reaction mixture was concentrated *in vacuo* to remove acetonitrile, and the aqueous phase was directly lyophilized to give the product **3-Na** (39.5 mg).

¹H NMR (400 MHz, CD₃OD) *δ* 6.83 (s, 1H), 4.48-4.36 (t, *J* = 8.8 Hz, 2H), 3.02-2.98 (t, *J* = 8.8 Hz, 2H), 1.88 (br, 1H), 1.69-1.64 (m, 1H), 1.29 (br, 1H), 0.72-0.67 (m, 1H), 0.28-0.22 (m, 1H), 0.20-0.16 (m, 1H).

### Example 4

### Step 1

Compound **1i** was resolved by preparative reversed-phase chromatography (column: Waters Atlantis T3 19 × 150 mm, 5 µm, mobile phase 1: 0.05% FA/H₂O; mobile phase 2: ACN) into **isomer 1i-1** (17 mg, 14% yield) and **isomer 1i-2** (52 mg, 42% yield).

### Compound 1i-1:

MS (ESI) m/z 455.5 [M+H]⁺
HPLC analysis: retention time 4.16 min, (column: Waters Acquity BEH C18 2.1 × 50 mm, 1.8 µm; mobile phase 1: 0.05% FA/H₂O; mobile phase 2: 0.05% FA/ACN).

### Compound 1i-2:

MS (ESI) m/z 455.5 [M+H]⁺
HPLC analysis: retention time 4.22 min, (column: Waters Acquity BEH C18 2.1 × 50 mm, 1.8 µm; mobile phase 1: 0.05% FA/H₂O; mobile phase 2: 0.05% FA/ACN).

### Step 2

Compound **1-1** was prepared according to the method of step 9 of Example 1 using compound **1i-2** as a reactant.

¹H NMR (400 MHz, DMSO-*d*₆) δ 6.85 (s, 1H), 4.18 (tq, *J* = 6.2, 3.6 Hz, 4H), 4.12 (d, *J* = 9.5 Hz, 1H), 2.16 (td, *J* = 7.9, 4.0 Hz, 1H), 1.24 (ddd, *J* = 10.8, 7.9, 3.3 Hz, 1H), 0.44 (ddd, *J* = 10.5, 8.1, 6.0 Hz, 1H), 0.22 (dt, *J* = 6.4, 3.7 Hz, 1H);
MS (ESI) m/z 263.3 [M+H]⁺

### Step 3

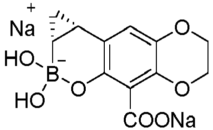

Compound **1-1** was added to a reaction flask, and 2.05 eq of an aqueous solution of NaOH was added. The solution was stirred for 1 h and then directly lyophilized to give compound **1-1-Na.**

¹H NMR (400 MHz, CD₃OD) δ 6.51 (s, 1H), 4.18-4.09 (m, 4H), 1.67-1.62 (m, 1H), 1.30 (br, 1H), 0.77-0.72 (m, 1H), 0.33-0.30 (m, 1H), 0.26-0.20 (m, 1H).

### Biological Evaluation

The present disclosure is further described and explained below with reference to test examples, which are not intended to limit the scope of the present disclosure.

### Test Example 1

### 1. Objective

To determine the minimum inhibitory concentrations (MIC) of compounds/compound combinations for 3 strains of drug-resistant bacteria.

### 2. Materials

1) The antibiotics cefepime and biapenem (purchased from MCE Inc.). The two antibiotics were diluted with corresponding solvents to form 12.8 mg/mL solutions.
2) QPX-7728 (synthesized according to WO2018005662A1), VNRX-5133 (synthesized according to WO2014089365A1), compound 1 and compound 2 were made into 3.2 mg/mL solutions in DMSO.
3) Test strains: *Escherichia coli* ARLG-2829 (Urine), which can be used to test the ability of β-lactamase inhibitors to potentiate the effect of cefepime on class B and class D β-lactamases; *Klebsiella pneumoniae* ATCC BAA-1705, which can be used to test the ability of β-lactamase inhibitors to potentiate the effect of cefepime and biapenem on class A β-lactamases; *Klebsiella pneumoniae* ATCC BAA-2472, which can be used to test the ability of β-lactamase inhibitors to potentiate the effect of cefepime and biapenem on class B β-lactamases.

### 3. Procedures

The biosafety cabinet was sterilized with ultraviolet light for 30 min. Glycerol tubes were taken out of a -80 °C freezer, and streaks were gently formed in the glycerol tubes using inoculating loops. Bacteria were inoculated onto MHIIA solid plates. The plates were incubated overnight in a 37 °C incubator.

30 µL of compound stock solution was added to column 1 of a 96-well v-bottom plate, and DMSO was added to columns 2-10. Subsequently, 15 µL was transferred from column 1 to column 2 using a pipette and well mixed gently, and then 15 µL of solution was transferred from column 2 to column 3; the serial dilution process proceeded to column 10 (200 × β-lactamase inhibitor working solutions were prepared). The two antibiotics were diluted with corresponding solvents to form 1.6 mg/mL solutions.

1 µL of 200× β-lactamase inhibitor solution and 1 µL of 1.6 mg/mL antibiotic were added to a new 96-well round-bottom plate. The plate that had been incubated overnight was taken out of the incubator, and several monoclones were picked into normal saline using an inoculating loop. The bacterial solution was adjusted to a concentration of about 1.0 × 10⁸CFU/mL and diluted 200-fold with medium, and 198 µL of the resulting bacterial solution was added to the test plate. The test plate was incubated in a 37 °C incubator for 18-24 h. The 96-well plate that had been incubated for 20 h was taken out of the incubator. The concentration that prevented visible bacterial growth was defined as the minimum inhibitory concentration.

The minimum inhibitory concentrations of compound 1 and compound 2 for three different strains in the presence of cefepime and biapenem are shown in the table below.

| **Minimum inhibitory concentration (µg/mL)** | | | | | |
|---|---|---|---|---|---|
| **Name of compound** | **Strain species** | | | | |
| | *Escherichia coli* ARLG-2829 | *Klebsiella pneumoniae* ATCC BAA-2472 | | *Klebsiella pneumoniae* ATCC BAA-1705 | |
| | Cefepime (8 ug/mL) | Cefepime (8 ug/mL) | Biapenem (8 ug/mL) | Cefepime (8 ug/mL) | Biapenem (8 ug/mL) |
| QPX-7728 | 1 | 16 | 8 | 0.25 | ≤0.03125 |
| Compound 1 | 0.5 | 16 | 4 | 0.5 | ≤0.03125 |
| VNRX-5133 | 2 | >16 | 2 | 0.25 | ≤0.03125 |
| Compound 2 | >16 | >16 | >16 | >16 | 1 |

### Test Example 2

### 1. Objective

To determine the minimum inhibitory concentrations (MIC) of compound 1-1-Na and QPX-7728 in combination with cefepime for drug-resistant bacteria.

### 2. Materials

1) The antibiotic cefepime was diluted with a corresponding solvent to form a 12.8 mg/mL solution.
2) QPX-7728 and compound 1-1-Na were made into 3.2 mg/mL solutions in DMSO.
3) Test strains: cefepime-resistant *Acinetobacter baumannii* (FDA-CDC AR-BANK#0033, FDA-CDC AR-BANK#0035, FDA-CDC AR-BANK#0078), cefepime-resistant *Escherichia coli* (FDA-CDC AR-BANK#0055, FDA-CDC AR-BANK#0114, FDA-CDC AR-BANK#0371, FDA-CDC AR-BANK#0370), cefepime-resistant *Klebsiella pneumoniae* (FDA-CDC AR-BANK#0003, FDA-CDC AR-BANK#0126, FDA-CDC AR-BANK#0080, FDA-CDC AR-BANK#0076, FDA-CDC AR-BANK#0158), and cefepime-resistant *Pseudomonas aeruginosa* (FDA-CDC AR-BANK#0439, FDA-CDC AR-BANK#0444, FDA-CDC AR-BANK#0246, FDA-CDC AR-BANK#0441), from Eurofins Inc.

### 3. Procedures

The biosafety cabinet was sterilized with ultraviolet light for 30 min. Glycerol tubes were taken out of a -80 °C freezer, and streaks were gently formed in the glycerol tubes using inoculating loops. Bacteria were inoculated onto MHIIA solid plates. The plates were incubated overnight in a 37 °C incubator.

30 µL of compound stock solution was added to column 1 of a 96-well v-bottom plate, and DMSO was added to columns 2-10. Subsequently, 15 µL was transferred from column 1 to column 2 using a pipette and well mixed gently, and then 15 µL of solution was transferred from column 2 to column 3; the serial dilution process proceeded to column 10 (200 × β-lactamase inhibitor working solutions were prepared). The antibiotic was diluted with a corresponding solvent to form a 1.6 mg/mL solution. The minimum inhibitory concentrations of QPX-7728 and compound 1-1-Na for three different strains in the presence of cefepime are shown in the table below.

| Strain | **Strain No.** | **Where the CPM MIC is fixed at 8 µg/mL, the concentration for achieving a bacteriostatic effect is** | | |
|---|---|---|---|---|
| | | CPM | QPX7728 | 1-1-Na |
| Cefepime-resistant *Acinetobacter baumannii* | FDA-CDC AR-BANK#0033 | >8 | >16 | >16 |
| | FDA-CDC AR-BANK#0035 | >8 | >16 | >16 |
| | FDA-CDC AR-BANK#0078 | >8 | 16 | >16 |
| Cefepime-resistant *Escherichia coli* | FDA-CDC AR-BANK#0055 | >8 | 0.5 | 1 |
| | FDA-CDC AR-BANK#0114 | >8 | ≤0.0156 | 0.03125 |
| | FDA-CDC AR-BANK#0371 | >8 | ≤0.0156 | ≤0.0156 |
| | FDA-CDC AR-BANK#0370 | >8 | 0.125 | 1 |
| Cefepime-resistant *Klebsiella pneumoniae* | FDA-CDC AR-BANK#0003 | >8 | 0.03125 | 0.125 |
| | FDA-CDC AR-BANK#0126 | >8 | ≤0.0156 | ≤0.0156 |
| | FDA-CDC AR-BANK#0080 | >8 | 0.5 | 2 |
| | FDA-CDC AR-BANK#0076 | >8 | 0.25 | 0.25 |
| | FDA-CDC AR-BANK#0158 | >8 | 0.5 | 2 |
| Cefepime-resistant *Pseudomonas aeruginosa* | FDA-CDC AR-BANK#0439 | >8 | >16 | >16 |
| | FDA-CDC AR-BANK#0444 | >8 | 4 | 16 |
| | FDA-CDC AR-BANK#0246 | >8 | >16 | >16 |
| | FDA-CDC AR-BANK#0441 | >8 | 2 | 8 |
| Control strain | *Escherichia coli* ATCC^{®} 25922 (0.016-0.12 µg/mL) | 0.0156 | --- | --- |
| Control strain | *Pseudomonas aeruginosa* ATCC^{®} 27853 (0.5∼4 µg/mL) | 1 | --- | --- |

The results show that both compound 1-1-Na and QPX-7728 can significantly restore to cefepime antibacterial activity against cefepime-resistant *Escherichia coli* and *Klebsiella pneumoniae.*

### Test Example 3

### 1. Objective

To compare the abilities of compound 1-1-Na and QPX7728 to restore bactericidal activity to CPM.

### 2. Materials

1) The antibiotic cefepime was diluted with a corresponding solvent to form a 12.8 mg/mL solution.
2) QPX-7728 and compound 1-1-Na were made into 3.2 mg/mL solutions in DMSO.
3) Test trains: cefepime-resistant *Klebsiella pneumoniae* (ARLG-1127 (Urine), ARLG-1195, ARLG-1196, ATCC BAA-1705, ATCC BAA-1898, ATCC BAA-1899, ATCC BAA-2343, ATCC BAA-2470) expressing different types of β-lactamases, from Eurofins Inc.

### 3. Procedures

The biosafety cabinet was sterilized with ultraviolet light for 30 min. Glycerol tubes were taken out of a -80 °C freezer, and streaks were gently formed in the glycerol tubes using inoculating loops. Bacteria were inoculated onto MHIIA solid plates. The plates were incubated overnight in a 37 °C incubator.

30 µL of compound stock solution was added to column 1 of a 96-well v-bottom plate, and DMSO was added to columns 2-10. Subsequently, 15 µL was transferred from column 1 to column 2 using a pipette and well mixed gently, and then 15 µL of solution was transferred from column 2 to column 3; the serial dilution process proceeded to column 10 (200 × β-lactamase inhibitor working solutions were prepared). The antibiotic was diluted with a corresponding solvent to form a 1.6 mg/mL solution. The minimum inhibitory concentrations of QPX-7728 and compound 1-1-Na for three different strains in the presence of cefepime are shown in the table below.

| Known β-lactamase | Class | Strain | Strain No. | MIC (ug/mL) | | | | | |
|---|---|---|---|---|---|---|---|---|---|
| | | | | CPM | Cefepime+1-1-Na (8 ug/ml) | Cefepime+QPX7728 (8 ug/ml) | 1-1-Na | QPX7728 | CPM |
| KPC,KPC-3,TEM,TEM-1 | Class A | Klebsiella pneumoniae | ARLG-1127 (Urine) | >128 | 0.25 | ≤0.125 | >128 | 64 | >128 |
| CTM-1 group, SHV, NDM | Class A,B | Klebsiella pneumoniae | ARLG-1195 | >128 | ≤0.125 | ≤0.125 | >128 | 32 | 64 |
| CTM-1 group, SHV, NDM,TEM | Class A,B | Klebsiella pneumoniae | ARLG-1196 | 128 | ≤0.125 | ≤0.125 | 128 | 32 | 128 |
| KPC-2,TEM,SHV | Class A | Klebsiella pneumoniae | ATCC BAA-1705 | >128 | ≤0.125 | ≤0.125 | >128 | 32 | 64 |
| KPC-2 | Class A | Klebsiella pneumoniae | ATCC BAA-1898 | >128 | ≤0.125 | ≤0.125 | >128 | 32 | 64 |
| KPC-2 | Class A | Klebsiella pneumoniae | ATCC BAA-1899 | >128 | ≤0.125 | ≤0.125 | 64 | 32 | 32 |
| KPC | Class A | Klebsiella pneumoniae | ATCC BAA-2343 | >128 | 0.25 | ≤0.125 | >128 | 32 | 128 |
| NDM-1 | Class B | Klebsiella pneumoniae | ATCC BAA-2470 | >128 | ≤0.125 | ≤0.125 | 64 | 16 | >128 |

The results show that both compound 1-1-Na and QPX-7728 can significantly restore to cefepime antibacterial activity against cefepime-resistant *Klebsiella pneumoniae.*

### Test Example 4

### 1. Objective

To compare the single intravenous administration exposures of compound 1-1-Na and the QPX7728 disodium salt in ICR mice.

### 2. Materials

The compound QPX-7728 disodium salt (synthesized according to CN109415386) and compound 1-1-Na (calculated amount of free drug = weighed amount × correction factor) were accurately weighed out, dissolved first in sterile water for injection to 40 mg/mL, and then diluted with normal saline to concentrations for administration. The solutions were prepared on the day of administration and stored at 4 °C.

### 3. Procedures

Single intravenous injections were administered to ICR mice, which were grouped as follows. Blood was collected from the saphenous vein or the fundus venous plexus at 0.083 h, 0.25 h, 0.5 h, 1 h, 2 h, 4 h, 6 h and 12 h after administration (about 0.1 mL/time point). A biological analysis method was developed to determine plasma concentrations at different time points, and related pharmacokinetic parameters were calculated.

| Group | Administration group | Dose mg/kg | Concentration mg/mL | Volume mL/kg | Animal No. | |
|---|---|---|---|---|---|---|
| | | | | | Female | Male |
| 1 | Compound 1-1- | 10 | 1 | 10 | 3F01∼3F03 | 3M01∼3M03 |
| 2 | Compound Na 1-1- | 100 | 10 | 10 | 4F01∼4F03 | 4M01∼4M03 |
| 3 | QPX7728 disodium salt | 10 | 1 | 10 | 1F01∼1F03 | 1M01∼1M03 |
| 4 | QPX7728 disodium salt | 100 | 10 | 10 | 2F01∼2F03 | 2M01∼2M03 |

The results are shown in the table below.

| **Sample** | **Dose (mg/kg)** | **t1/2z (h)** | **Tmax (h)** | **Cmax (ng/mL)** | **AUC0-t (h*ng/mL)** | **AUCinf (h*ng/mL)** |
|---|---|---|---|---|---|---|
| **1-1-Na** | 10 | 1.92 | 0.083 | 146333.33 | 210877.57 | 213340.25 |
| | 100 | 2.96 | 0.083 | 607000.00 | 1395153.66 | 1539755.27 |
| **QPX7728** disodium salt | 10 | 2.90 | 0.083 | 65800.00 | 44689.03 | 46044.19 |
| | 100 | 2.1 | 0.083 | 399000.0 | 297241.4 | 299678.7 |

The result shows that the single intravenous administration exposure of compound 1-1-Na in mice was about 5 times that of the QPX7728 disodium salt. Therefore, a similar drug effect can be produced at a reduced dose that causes fewer toxic and side effects.

## Claims

1. A compound of formula (I) or a pharmaceutically acceptable salt thereof, or a stereoisomer, rotamer, tautomer or deuterated compound thereof, 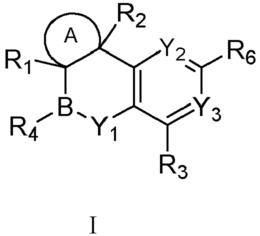 wherein,
ring A is selected from the group consisting of the following optionally substituted ring systems: a carbocycle, a heterocycle, an aromatic ring and a heteroaromatic ring;
Y₁ is selected from the group consisting of -O- and -S-;
Y₂ is selected from the group consisting of CR₅ and N, Y₃ is selected from the group consisting of CR₅' and N, and Y₂ and Y₃ are not simultaneously N;
R₁ and R₂ are each independently selected from the group consisting of hydrogen and the following optionally substituted groups: alkyl, alkenyl, alkynyl, halogen, deuterium, hydroxy, sulfhydryl, - NRiRⱼ, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, alkoxy, alkylthioether group, cycloalkyl, heterocyclyl, aryl and heteroaryl;
R₃ is independently selected from the group consisting of hydrogen and the following optionally substituted groups: alkyl, alkenyl, alkynyl, halogen, deuterium, hydroxy, sulfhydryl, -NRᵢRⱼ, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, alkoxy, alkylthioether group and carboxylic acid isostere;
R₄ is independently selected from the group consisting of the following optionally substituted groups: -NRᵢRⱼ, hydroxy, alkoxy and halogen;
R₅ is selected from the group consisting of hydrogen and the following optionally substituted groups: alkyl, alkenyl, alkynyl, halogen, deuterium, hydroxy, sulfhydryl, -NRᵢRⱼ, -C(O)Rₖ, -C(O)ORₖ, - S(O)Rₖ, -S(O)ORₖ, -S(O)(O)Rₖ, -S(O)(O)ORₖ, -C(S)Rₖ, nitro, cyano, alkoxy, alkylthioether group, cycloalkyl, heterocyclyl, aryl and heteroaryl, or R₅ and R₆, together with their adjacent carbon atoms, form the following optionally substituted ring system: a carbocycle, a heterocycle, an aromatic ring, a heteroaromatic ring, a spirocarbocycle, a spiroheterocycle, a fused carbocycle, a fused heterocycle, a fused aromatic ring or a fused heteroaromatic ring;
R₅' is selected from the group consisting of hydrogen and the following optionally substituted groups: alkyl, alkenyl, alkynyl, halogen, deuterium, hydroxy, sulfhydryl, -NRᵢRⱼ, -C(O)Rₖ, -C(O)ORₖ, - S(O)Rₖ, -S(O)ORₖ, -S(O)(O)Rₖ, -S(O)(O)ORₖ, -C(S)Rₖ, nitro, cyano, alkoxy, alkylthioether group, cycloalkyl, heterocyclyl, aryl and heteroaryl, or R₅' and R₆, together with their adjacent carbon atoms, form the following optionally substituted ring system: a carbocycle, a heterocycle, an aromatic ring, a heteroaromatic ring, a spirocarbocycle, a spiroheterocycle, a fused carbocycle, a fused heterocycle, a fused aromatic ring or a fused heteroaromatic ring;
provided that R₆, at least one of R₅ and R₅', and adjacent carbon atoms form a ring system;
Rᵢ and Rⱼ are each independently selected from the group consisting of hydrogen atom, hydroxy, C₁-C₆ alkyl and C₁-C₆ alkoxy;
Rₖ is independently selected from the group consisting of hydrogen atom, alkyl, haloalkyl, alkoxy, hydroxy and -NRᵢRⱼ, wherein the alkyl, alkoxy and haloalkyl are optionally substituted with one or more substituents selected from the group consisting of alkyl, halogen, hydroxy, sulfhydryl, -NRᵢRⱼ, oxo, thio, carboxyl, nitro, cyano, alkoxy, alkylthioether group, alkenyl, alkynyl, cycloalkyl, heterocyclyl, aryl and heteroaryl.

2. The compound according to claim 1, wherein the compound of formula I is a compound of formula 1-1 or I-2, 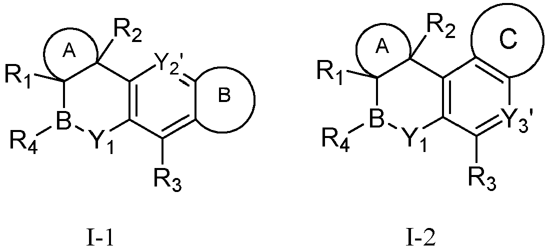
wherein ring A, R₁, R₂, R₃, R₄ and Y₁ are as described in claim 1;
ring B and ring C are each independently selected from the group consisting of the following optionally substituted ring systems: a carbocycle, a heterocycle, an aromatic ring, a heteroaromatic ring, a spirocarbocycle, a spiroheterocycle, a fused carbocycle, a fused heterocycle, a fused aromatic ring and a fused heteroaromatic ring;
Y₂' is selected from the group consisting of CR₅ and N;
Y₃' is selected from the group consisting of CR₅' and N;
R₅ and R₅' are each independently selected from the group consisting of hydrogen and the following optionally substituted groups: alkyl, alkenyl, alkynyl, halogen, deuterium, hydroxy, sulfhydryl, - NRiRⱼ, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, alkoxy, alkylthioether group, cycloalkyl, heterocyclyl, aryl and heteroaryl.

3. The compound according to claim 1, wherein the ring system formed from R₆, at least one of R₅ and R₅' and adjacent carbon atoms is selected from the group consisting of the following ring systems: 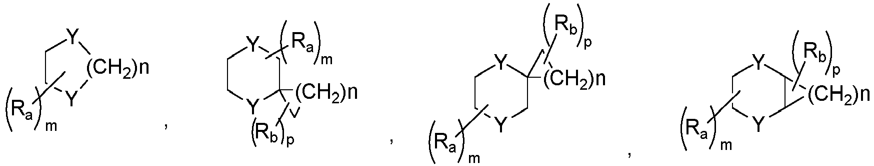 wherein,
Y is independently selected from the group consisting of CH₂, NH, O and S;
Rₐ and R_{b} are each independently selected from the group consisting of the following optionally substituted groups: C₁-C₆ alkyl, halogen, deuterium, hydroxy, sulfhydryl, -NRᵢRⱼ, oxo, thio, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthioether group, C₂-C₆ alkenyl, C₂-C₆ alkynyl, 3- to 6-membered cycloalkyl, 3- to 6-membered heterocyclyl, 7- to 10-membered fused cycloalkyl, 7- to 10-membered fused heterocyclyl, 6- to 10-membered aryl, 5- to 10-membered heteroaryl, 8- to 12-membered fused cycloaryl, and 5-to 12-membered fused heteroaryl;
n is selected from the group consisting of 1, 2, 3, 4, 5 and 6;
m and p are each independently selected from the group consisting of 0, 1, 2, 3, 4, 5 and 6.

4. The compound according to claim 2, wherein ring B and ring C are each independently selected from the group consisting of the following ring systems: 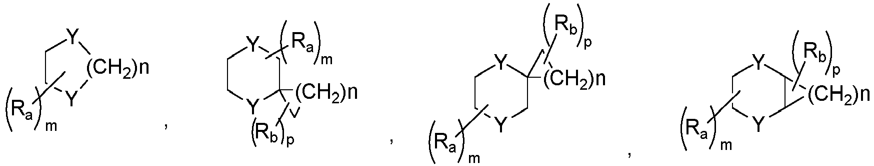 wherein,
Y is independently selected from the group consisting of CH₂, NH, O and S;
Rₐ and R_{b} are each independently selected from the group consisting of the following optionally substituted groups: C₁-C₆ alkyl, halogen, deuterium, hydroxy, sulfhydryl, -NRᵢRⱼ, oxo, thio, -C(O)Rₖ, -C(O)ORₖ, -S(O)Rₖ, -S(O)ORₖ, -S(O)(O)Rₖ, -S(O)(O)ORₖ, -C(S)Rₖ, nitro, cyano, C₁-C₆ alkoxy, C₁-C₆ alkylthioether group, C₂-C₆ alkenyl, C₂-C₆ alkynyl, 3- to 6-membered cycloalkyl, 3- to 6-membered heterocyclyl, 7- to 10-membered fused cycloalkyl, 7- to 10-membered fused heterocyclyl, 6- to 10-membered aryl, 5- to 10-membered heteroaryl, 8- to 12-membered fused cycloaryl, and 5-to 12-membered fused heteroaryl;
n is selected from the group consisting of 1, 2, 3, 4, 5 and 6;
m and p are each independently selected from the group consisting of 0, 1, 2, 3, 4, 5 and 6.

5. The compound according to any one of claims 1-4, wherein R₁ and R₂ are each independently selected from the group consisting of hydrogen and the following optionally substituted groups: C₁-C₆ alkyl, C₂-C₆ alkenyl, C₂-C₆ alkynyl, halogen, deuterium, hydroxy, -NRᵢRⱼ and C₁-C₆ alkoxy.

6. The compound according to any one of claims 1-4, wherein ring A is selected from the group consisting of optionally substituted 3- to 12-membered, preferably 3- to 6-membered, carbocycle and heterocycle.

7. The compound according to any one of claims 1-4, wherein R₃ is independently selected from the group consisting of optionally substituted -C(O)ORₖ and an optionally substituted carboxylic acid isostere.

8. The compound according to claim 1, being selected from the group consisting of 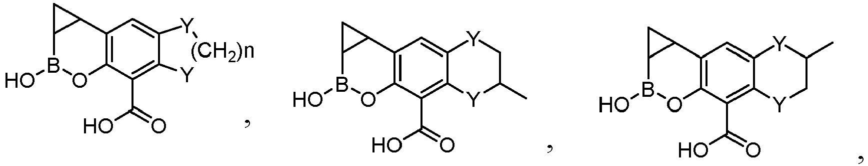 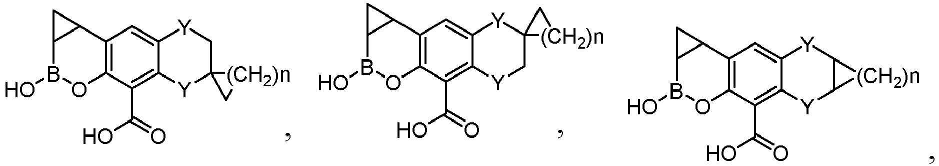 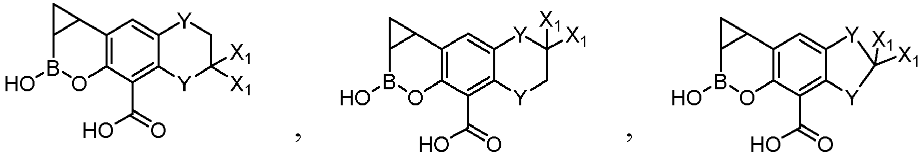
or pharmaceutically acceptable salts thereof, or stereoisomers, rotamers, tautomers or deuterated compounds thereof, wherein,
Y is independently selected from the group consisting of CH₂, NH, O and S; n is independently selected from the group consisting of 1, 2, 3, 4, 5 and 6; X₁ is independently selected from the group consisting of F, Cl and Br.

9. The compound according to claim 1, being selected from the group consisting of 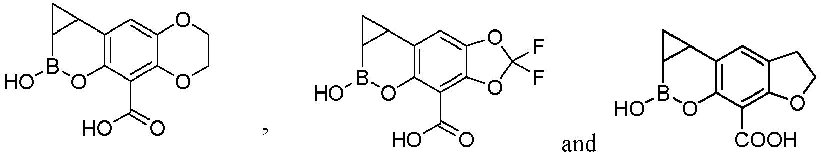 or pharmaceutically acceptable salts thereof, or stereoisomers, rotamers, tautomers or deuterated compounds thereof.

10. The compound according to claim 1, wherein the compound is selected from the group consisting of 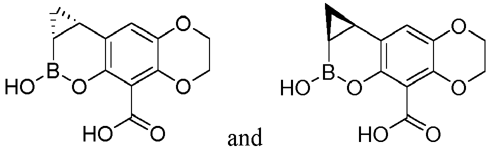 or pharmaceutically acceptable salts thereof, or stereoisomers, rotamers, tautomers or deuterated compounds thereof, preferably 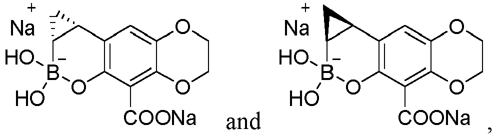 or stereoisomers, rotamers, tautomers or deuterated compounds thereof.

11. A pharmaceutical composition comprising at least one of the compounds or the pharmaceutically acceptable salts thereof, or the stereoisomers, rotamers, tautomers or deuterated compounds thereof according to any one of claims 1-10, and a pharmaceutically acceptable carrier, diluent or excipient.

12. Use of the compound or the pharmaceutically acceptable salt thereof, or the stereoisomer, rotamer, tautomer or deuterated compound thereof according to any one of claims 1-10 in the preparation of a medicament for treating a bacterial infection.

13. The use according to claim 12, wherein the infection comprises a bacterium selected from the group consisting of: *Pseudomonas acidovorans, Pseudomonas alcaligenes, Pseudomonas putida, Burkholderia cepacia, Aeromonas hydrophilia, Francisella tularensis, Morganella morganii, Proteus mirabilis, Proteus vulgaris, Providencia alcalifaciens, Providencia rettgeri, Providencia stuartii, Acinetobacter baumannii, Bordetella pertussis, Bordetella para pertussis, Bordetella bronchiseptica, Haemophilus ducreyi, Pasteurella multocida, Pasteurella haemolytica, Branhamella catarrhalis, Borrelia burgdorferi, Kingella, Gardnerella vaginalis, Bacteroides distasonis, Bacteroides* 3452A homology group, *Clostridium difficile, Mycobacterium tuberculosis, Mycobacterium avium, Mycobacterium intracellulare, Mycobacterium leprae, Corynebacterium diphtheriae, Corynebacteriumulcerans, Streptococcus pneumoniae, Streptococcusagalactiae, Streptococcus pyogenes, Enterococcusfaecalis, Enterococcus faecium, Staphylococcusaureus, Staphylococcus epidermidis, Staphylococcus saprophyticus, Staphylococcus intermedius, Staphylococcus hyicus subsp. hyicus, Staphylococcus haemolyticus, Staphylococcus hominis* and *Staphylococcus saccharolyticus.*

14. The use according to claim 12, wherein the infection comprises a bacterium selected from the group consisting of: *Pseudomonas aeruginosa, Pseudomonas fluorescens, Stenotrophomonas maltophilia, Escherichia coli, Citrobacter freundii, Salmonella typhimurium, Salmonella typhi, Salmonella paratyphi, Salmonella enteritidis, Shigella dysenteriae, Shigellaflexneri, Shigella sonnei, Enterobacter cloacae, Enterobacter aerogenes, Klebsiella pneumoniae, Klebsiella oxytoca, Serratia marcescens, Acinetobacter calcoaceticus, Acinetobacter haemolyticus, Yersinia enterocolitica, Yersinia pestis, Yersinia pseudotuberculosis, Yersinia intermedia, Haemophilus influenzae, Haemophilus parainfluenzae, Haemophilus haemolyticus, Haemophilusparahaemolyticus, Helicobacter pylori, Campylobacterfetus, Campylobacter jejuni, Campylobacter coli, Vibrio cholerae, Vibrio parahaemolyticus, Legionella pneumophila, Listeria monocytogenes, Neisseria gonorrhoeae, Neisseria meningitidis, Moraxella, Bacteroides fragilis, Bacteroidesvulgatus, Bacteroides ovatus, Bacteroidesthetaiotaomicron, Bacteroides uniformis, Bacteroideseggerthii* and *Bacteroides splanchnicus.*
